# EUROPÄISCHE PATENTSCHRIFT

(11) **EP 0 944 582 B1**
(45) Veröffentlichungstag und Bekanntmachung des Hinweises auf die Patenterteilung: **02.07.2003**
(21) Anmeldenummer: 97953714.9
(22) Anmeldetag: 28.11.1997
(51) Int. Cl.: C07C 233/87, C07C 311/21, C07D 295/12, C07D 295/02, C07D 215/36, C07D 241/42

(54) **KETOBENZAMIDE ALS CALPAIN-INHIBITOREN**
KETOBENZAMIDES AS CALPAIN INHIBITORS
CETOBENZAMIDES S'UTILISANT COMME INHIBITEURS DE LA CALPAINE

(30) Priorität: 11.12.1996 DE 19651316
(43) Veröffentlichungstag der Anmeldung: 29.09.1999
(73) Patentinhaber: Abbott GmbH & Co. KG, 65205 Wiesbaden (DE)
(72) Erfinder: LUBISCH, Wilfried, D-68159 Mannheim (DE); MÖLLER, Achim, D-67269 Grünstadt (DE); TREIBER, Hans-Jörg, D-68782 Brühl (DE)
(74) Vertreter: Goldbach, Klara, Dr.
(86) Internationale Anmeldenummer: EP9706655
(87) Internationale Veröffentlichungsnummer: WO98025883

(56) Entgegenhaltungen:
- EP-A- 0 520 336
- WO-A-92/11850
- ANGELASTRO ET AL.: "Alpha-Diketones and alpha-Keto Ester Derivatives" J. MED. CHEM., Bd. 33, Nr. 1, 1990, Seiten 11-13, XP002058114 in der Anmeldung erwähnt

## Beschreibung

Die vorliegende Erfindung betrifft neuartige Ketobenzamide, deren Herstellung sowie deren Verwendung bei der Bekämpfung von Krankheiten.

Calpaine stellen intracelluläre, proteolytische Enzyme aus der Gruppe der sogenannten Cystein-Proteasen dar und werden in vielen Zellen gefunden. Calpaine werden durch erhöhte Kalziumkonzentration aktiviert, wobei man zwischen Calpain I oder µ-Calpain, das durch µ-molare Konzentrationen von Kalzium-Ionen akiviert wird, und Calpain II oder m-Calpain, das durch m-molare Konzentrationen von Kalzium-Ionen aktiviert wird, unterscheidet (P.Johnson, Int.J.Biochem. 1990, 22(8), 811-22). Heute werden noch weitere Calpain-Isoenzyme postuliert (K.Suzuki et al., Biol.Chem. Hoppe-Seyler, 1995, 376(9),523-9).

Man vermutet, daß Calpaine in verschiedenen physiologischen Prozessen eine wichtige Rolle spielen. Dazu gehören Spaltungen von regulatorischen Proteinen wie Protein-Kinase C, Cytoskelett-Proteine wie MAP 2 und Spektrin, Muskelproteine, Proteinabbau in rheumatoider Arthritis, Proteine bei der Aktivierung von Plättchen, Neuropeptid-Metabolismus, Proteine in der Mitose und weitere, die in. M.J.Barrett et al., Life Sci. 1991, 48, 1659-69 und K.K.Wang et al., Trends in Pharmacol.Sci., 1994, 15, 412-9 aufgeführt sind.

Bei verschiedenen pathophysiologischen Prozessen wurden erhöhte Calpain-Spiegel gemessen, zum Beispiel: Ischämien des Herzens (z.B. Herzinfarkt), der Niere oder des Zentralnervensystems (z.B. "Stroke"), Entzündungen, Muskeldystrophien, Katarakten der Augen, Verletzungen des Zentralnervensystems ( z.B.Trauma), Alzheimer Krankheit usw.(siehe K.K. Wang, oben). Man vermutet einen Zusammenhang dieser Krankheiten mit erhöhten und anhaltenden intrazellulären Kalziumspiegeln. Dadurch werden Kalzium-abhängige Prozesse überaktiviert und unterliegen nicht mehr der physiologischen Regelung. Dementsprechend kann eine Überaktivierung von Calpainen auch pathophysiologische Prozesse auslösen.

Daher wurde postuliert, daß Inhibitoren der Calpain-Enzyme für die Behandlung dieser Krankheiten nützlich sein können. Verschiedene Untersuchungen bestätigen dies. So haben Seung-Chyul Hong et al., Stroke 1994, 25(3), 663-9 und R.T.Bartus et al., Neurological Res. 1995, 17, 249-58 eine neuroprotektive Wirkung von Calpain-Inhibitoren in akuten neurodegenerativen Störungen oder Ischämien, wie sie nach Hirnschlag auftreten, gezeigt. Nach experimentellen Gehirntraumata verbesserten Calpain-Inhibitoren die Erholung der auftretenden Gedächtnisleistungsdefizite und neuromotrischen Störungen (K.E.Saatman et al. Proc.Natl.Acad.Sci. USA, 1996, 93,3428-3433). C.L.Edelstein et al., Proc.Natl.Acad.Sci. USA, 1995, 92, 7662-6, fanden eine protektive Wirkung von Calpain-Inhibitoren auf durch Hypoxie geschädigte Nieren. Yoshida, Ken Ischi et al., Jap.Circ.J. 1995, 59(1), 40-8, konnten günstige Effekte von Calpain-Inhibitoren nach cardialen Schädigungen aufzeigen, die durch Ischämie oder Reperfusion erzeugt wurden. Da Calpain-Inhibitoren die Freisetzung des β-AP4-Proteins hemmen, wurde eine potentielle Anwendung als Therapeutikum der Alzheimer Krankheit vorgeschlagen (J.Higaki et al., Neuron, 1995, 14, 651-59). Die Freisetzung von Interleukin-1α wird ebenfalls durch Calpain-Inhibitoren gehemmt (N.Watanabe et al., Cytokine 1994, 6(6), 597-601). Weiterhin wurde gefunden, daß Calpain-Inhibitoren cytotoxische Effekte an Tumorzellen zeigen ( E.Shiba et al. 20th Meeting Int.Ass.Breast Cancer Res., Sendai Jp, 1994, 25.-28.Sept., Int.J.Oncol. 5(Suppl.), 1994, 381).

Weitere mögliche Anwendungen von Calpain-Inhibitoren sind in K.K.Wang, Trends in Pharmacol.Sci., 1994, 15, 412-8, aufgeführt.

Calpain-Inhibitoren sind in der Literatur bereits beschrieben worden. Überwiegend sind dies jedoch entweder irreversible oder peptidische Inhibitoren. Irreversible Inhibitoren sind in der Regel alkylierende Substanzen und haben den Nachteil, daß sie im Organismus unselektiv reagieren oder instabil sind. So zeigen diese Inhibitoren oft unerwünschte Nebeneffekte, wie Toxizität, und sind dadurch in der Anwendung eingeschränkt oder nicht brauchbar. Zu den irreveriblen Inhibitoren kann man zum Beispiel die Epoxide E 64 (E.B.McGowan et al., Biochem.Biophys.Res.Commun. 1989, 158, 432-5), α-Halogenketone ( H.Angliker et al., J.Med.Chem. 1992, 35, 216-20) und Disulfide (R.Matsueda et al., Chem.Lett. 1990, 191-194) zählen.

Viele bekannte reversible Inhibitoren von Cystein-Proteasen wie Calpain stellen peptidische Aldehyde dar, insbesondere dipeptidische und tripepidische Aldehyde wie zum Beispiel Z-Val-Phe-H (MDL 28170) (S.Mehdi, Tends in Biol.Sci. 1991, 16, 150-3) und die Verbindungen aus EP 520336. Unter physiologischen Bedingungen haben peptidische Aldehyde häufig den Nachteil, daß sie auf Grund der großen Reaktivität instabil sind, schnell metabolisiert werden können und zu unspezifischen Reaktionen neigen, die die Ursache von toxischen Effekten sein können (J.A.Fehrentz und B.Castro, Synthesis 1983, 676-78). Die Verwendung von peptidischen Aldehyden in der Behandlung von Krankheiten ist somit nur eingeschränkt möglich oder nicht sinnvoll.

Einen Fortschritt stellt die Entdeckung dar, daß bestimmte peptidische Keton-Derivate ebenfalls Inhibitoren von Cystein-Proteasen und insbesondere Calpain darstellen. So sind zum Beispiel bei Serin-Proteasen Keton-Derivate als Inhibitoren bekannt, wobei die Keto-Gruppe von einer elektronenziehenden Gruppe wie CF₃ aktiviert wird. Bei Cystein-Proteasen sind Derivate mit durch CF₃ oder ähnlichen Gruppen aktivierte Ketone wenig oder nicht wirksam (M.R.Angelastro et al., J.Med.Chem. 1990,33, 11-13). Überraschenderweise konnten bei Calpain bisher nur Keton-Derivate, bei denen einerseits α-ständige Abgangsgruppen eine irreversible Hemmung verursachen und andererseits ein Carbonsäure-Derivat die Keto-Gruppe aktiviert, als wirksame Inhibitoren gefunden werden (siehe M.R.Angelastro et al., siehe oben; WO 92/11850; WO 92,12140; WO 94/00095 und WO 95/00535). Jedoch sind von diesen Ketoamiden und Ketoestern bisher nur peptidische Derivate als wirksam beschrieben worden (Zhao Zhao Li et al., J.Med.Chem. 1993, 36, 3472-80; S.L.Harbenson et al., J.Med.Chem. 1994, 37, 2918-29 und siehe oben M.R.Angelastro et al.).

Weiter sind Ketobenzamide aus der Literatur bekannt. So wurde der Ketoester PhCO-Abu-COOCH₂CH₃ in WO 91/09801, WO 94/00095 und 92/11850 beschrieben. Das analoge Phenyl-Derivat Ph-CONH-CH(CH₂Ph)-CO-COOCH₃ wurde in M.R.Angelastro et al., J.Med.Chem. 1990,33, 11-13 als jedoch nur schwacher Calpain-Inhibitor gefunden. Dieses Derivat ist auch in J.P.Burkhardt, Tetrahedron Lett., 1988, 3433-36 beschrieben. Die Bedeutung der substituierten Benzamide ist jedoch bisher nie untersucht worden.

Es wurden nun substituierte nicht-peptidische Ketobenzamid-Derivate mit einer verbesserten Wirkung gefunden.

Gegenstand der vorliegenden Erfindung sind Ketobenzamide der Formel I 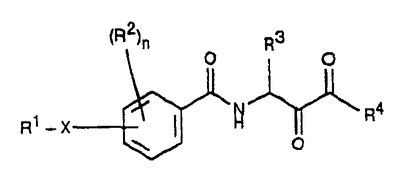 und deren tautomere und isomere Formen sowie gegebenenfalls deren physiologisch verträgliche Salze, worin die Variablen folgende Bedeutung haben:
- R¹: Phenyl, Naphthyl, Chinolyl, Pyridyl, Pyrimidyl, Pyrazyl, Pyridazyl, Chinazolyl, Chinoxalyl, Thienyl, Benzothienyl, Benzofuryl,Benzimidazolyl, Furanyl, Indolyl, Isochinolin, Tetrahydroisochinolin oder Tetrahydrochinolin, wobei die aromatischen und heteroaromatischen Ringe noch durch ein, zwei oder drei Reste R⁵ substituiert sein können,
- R²: Chlor, Brom, Fluor, C₁-C₆-Alkyl, C₂-C₆-Alkenyl, C₂-C₆-Alkinyl, C₁-C₆-Alkyl-Phenyl, C₂-C₆-Alkenyl-Phenyl, C₂-C₆-Alkinyl-Phenyl, Phenyl, NHCO-C₁-C₄-Alkyl, -NHCO-Phenyl, -NHCO-Naphthyl, H₂N-SO₂-C₁₋₄-Alkyl-, COOH, -COO-C₁₋₄-Alkyl, -CONH-C₁₋₄-Alkyl, C₁₋₄-Alkoxy, NO₂, oder NH₂,
- R³: C₁-C₆-Alkyl, das noch einen Phenyl-, Cyclopropyl-, Cyclobutyl-, Cyclopentyl-, Cyclohexyl-, Cycloheptyl, Indolyl-, Pyridyloder Naphthyl-Ring tragen kann, der seinerseits mit ein oder zwei Resten R⁵ substituiert sein kann,
- X: eine Bindung, -(CH₂)ₘ -, -(CH₂)ₘ -O-(CH₂)ₒ -, -(CH₂)ₙ-S-(CH₂)ₘ-, -(CH₂)ₙ-SO-(CH₂)ₘ -, -(CH₂)ₙ-SO₂-(CH₂)ₘ-, -CH=CH-, -C≡C-, -CO-CH=CH-, CO-(CH₂)ₘ-, -(CH₂)ₘ-NHCO-(CH₂)ₒ-, - (CH₂)ₘ-CONH-(CH₂)ₒ-, -(CH₂)ₘ-NHSO₂- (CH₂)ₒ-, -NH-CO-CH=CH-, -CH=CH-CO-NH-, -(CH₂)ₘ-SO₂NH-(CH₂)ₒ- oder 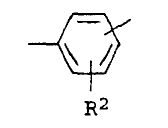
- R⁴: OR⁶, NR⁷R⁸, 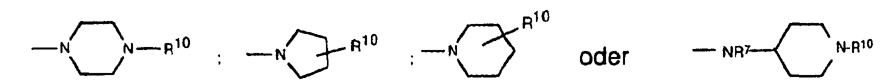
- R⁵: Wasserstoff, C₁-C₄-Alkyl, -O-C₁-C₄-Alkyl, OH, Cl, F, Br, J, CF₃, NO₂, NH₂, CN, COOH, COO-C₁-C₄-Alkyl, -NHCO-C₁-C₄-Alkyl, -NHCO-Phenyl, -NHSO₂-C₁-C₄-Alkyl, -NHSO₂-Phenyl, -SO₂-C₁-C₄-Alkyl oder -SO₂-Phenyl,
- R⁶: Wasserstoff oder C₁-C₆-Alkyl, das durch einem Phenylring substituiert kann, der selbst noch durch einen oder zwei Reste R⁹ substituiert sein kann,
- R⁷: Wasserstoff oder C₁-C₆-Alkyl,
- R⁸: Wasserstoff oder C₁-C₆-Alkyl, das noch durch einem Phenylring, der einen oder zwei Reste R⁹ tragen kann, oder einen der Reste 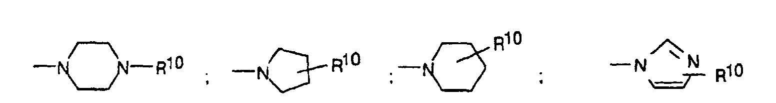 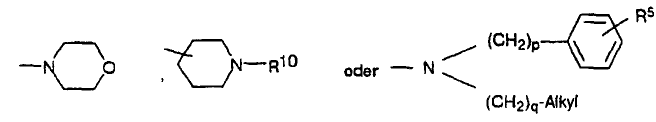 (p,q=0,1,2,3 oder 4) substituiert sein kann,
- R⁹: Wasserstoff, C₁-C₄-Alkyl, -O-C₁-C₄-Alkyl, OH Cl, F, Br, J, CF₃, NO₂, NH₂, CN, COOH, COO-C₁-C₄-Alkyl, -NHCO-C₁-C₄-Alkyl, -NHCO-Phenyl, -NHSO₂-C₁-C₄-Alkyl, -NHSO₂-Phenyl, -SO₂-C₁-C₄-Alkyl oder -SO₂-Phenyl,
- R¹⁰: Wasserstoff oder C₁-C₆-Alkyl, das durch einen Phenylring substituiert kann, der noch durch einen oder zwei Reste R⁹ substituiert sein kann,
- n: die Zahl 0, 1 oder 2,
- m: die Zahl 0, 1, 2, 3 oder 4 und
- o: die Zahl 0, 1, 2, 3 oder 4.

Bevorzugt sind die Verbindungen der Formel I, worin
- R²: Wasserstoff, C₁-C₄-Alkyl, Fluor oder Chlor,
- R³: -CH₂-Phenyl, -CH₂-Cyclohexyl, n-Butanyl oder n-Pentanyl, die jeweils durch einen Rest R⁵ substituiert sein können,
- R⁴: -NR⁸ bedeuten und
- R¹,: X und n die in Anspruch 1 angegebenen Bedeutungen haben.

Die Verbindungen der Formel I können als Racemate oder als enantiomerenreine Verbindungen oder als Diastereomere eingesetzt werden. Werden enantiomerereine Verbindungen gewünscht, kann man diese beispielweise dadurch erhalten, daß man mit einer geeigneten optisch aktiven Base oder Säure eine klassische Racematspaltung mit den Verbindungen der Formel I oder ihren Zwischenprodukten durchführt. Die enantiomeren Verbindungen können ebenfalls durch Einsatz von kommerziell erhältlichen Verbindungen, zum Beispiel optisch aktiven Aminosäuren wie Phenylalanin, Tryptophan und Tyrosin, hergestellt werden.

Gegenstand der Erfindung sind auch zu Verbindungen der Formel I mesomere oder tautomere Verbindungen, beispielweise solche, bei denen die Ketogruppe der Formel I als Enol-Tautomeres vorliegt.

Ein Teil der neuen Verbindungen I kann eine basische oder saure Gruppe enthalten. In diesen Fällen können die Verbindungen I in Form ihrer physiologisch verträglichen Salze vorliegen, die sich durch Umsatz der Verbindungen I mit einer geeigneten Säure oder Base erhalten lassen.

Geeignete Säuren zur Salzbildung mit erfindungsgemäßen Verbindungen I, die eine basische Gruppe enthalten, sind zum Beispiel Salzsäure, Citronensäure, Weinsäure, Milchsäure, Phosphorsäure, Methansulfonsäure, Essigsäure, Ameisensäure, Maleinsäure, Fumarsäure, Äpfelsäure, Bernsteinsäure, Malonsäure und Schwefelsäure. Geeignete Basen sind zum Beispiel Kaliumhydroxid, Natriumhydroxid, Lithiumhydroxid, Triethylamin, α,α,α-Tris-(hydroxymethyl)methylamin und andere Amine.

Die Herstellung der erfindungsgemäßen Ketobenzamide I kann auf verschiedenen Wegen erfolgen, die in den Syntheseschemata 1 und 2 skizziert sind.

Die Karbonsäureester II werden mit Säuren oder Basen wie Salzsäure, Lithiumhydroxid, Natriumhydroxid oder Kaliumhydroxid in wäßrigen Medium oder in Gemischen aus Wasser und organischen Lösungsmitteln wie Alkoholen oder Tetrahydrofuran bei Raumtemperatur oder erhöhten Temperaturen, wie 25-100°C, in die Säuren III überführt. Die Säuren III werden mit einem α-Aminosäure-Derivat verknüpft,wobei man übliche Bedingungen benutzt, die zum Beispiel im Houben-Weyl, Methoden der organischen Chemie, 4.Aufl., E5, Kap. V, und C.R.Larock, Comprehensive Organic Transformations, VCH Publisher, 1989, Ch.9 aufgelistet sind.

Die Carbonsäure III wird in ein "aktiviertes" Säure-Derivat R¹-L, wobei L eine Abgangsgruppe wie Cl, Imidazol und N-Hydroxybenzotriazol darstellt, umgewandelt und durch Umsatz mit einem Aminosäure-Derivat H₂N-CHR³-COOR in das Derivat IV überführt. Diese Reaktion erfolgt in wasserfreien, inerten Lösungsmitteln wie Methylenchlorid, Tetrahydrofuran und Dimethylformamid bei Temperaturen von -20 bis +25°C.

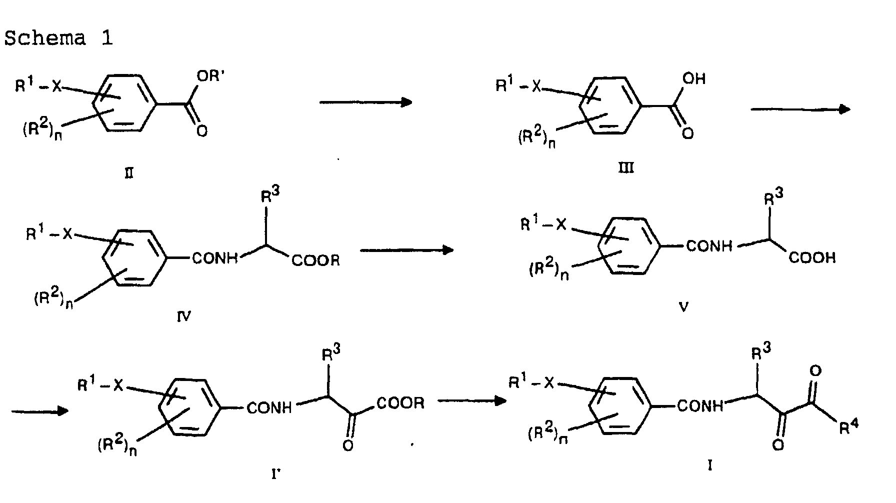

Die Derivate IV, die in der Regel Ester darstellen, werden analog der oben beschriebenen Hydrolyse in die Ketokarbonsäuren V überführt. In einer Dakin-West analogen Reaktion werden die Ketoester I' hergestellt, wobei nach einer Methode von Zhao Zhao Li et al.. J.Med.Chem., 1993, 36, 3472-80 gearbeitet wird. Dabei wird eine Karbonsäure wie V bei erhöhter Temperatur (50-100°C) in Lösungsmitteln, wie zum Beispiel Tetrahydrofuran, mit Oxalsäuremonoesterchlorid umgesetzt und anschließend das so erhaltene Produkt mit Basen wie Natriumethanolat in Ethanol bei Temperaturen von 25-80°C zum erfindungsgemäßen Ketoester I' umgesetzt. Die Ketoester I' können, wie oben beschrieben, zum Beispiel zu den erfindungsgemäßen Ketocarbonsäuren hydrolysiert werden.

Die Umsetzung zu Ketobenzamiden I' erfolgt ebenfalls analog der Methode von Zhao Zhao Li et al. (siehe oben). Die Ketogruppe in I' wird durch Zugabe von 1,2-Ethandithiol unter Lewissäure-Katalyse, zum Beispiel mit Bortrifluoridetherat, in inerten Lösungsmitteln, wie Methylenchlorid, bei Raumtemperatur geschützt, wobei ein Dithian anfällt. Diese Derivate werden mit Aminen R⁴-H in polaren Lösungsmitteln, wie Alkoholen, bei Temperaturen von 0-80°C umgesetzt, wobei die Ketoamide I (z.B. R⁴ = NR⁷R⁸) anfallen.

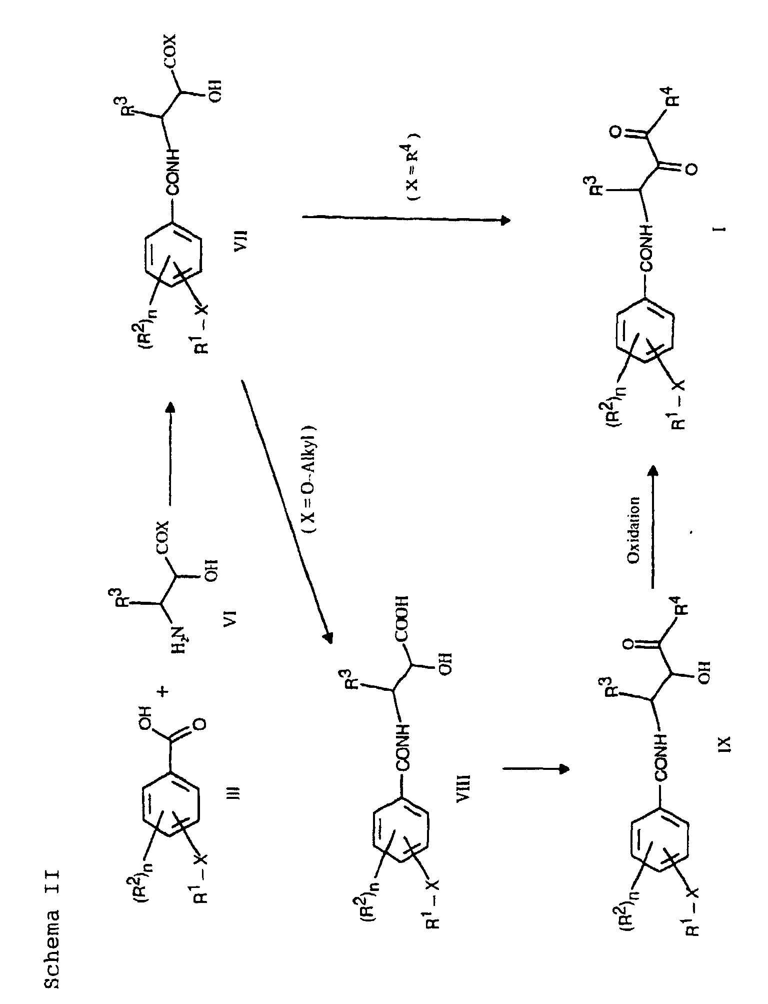

Eine alternative Methode ist im Schema 2 dargestellt. Die Ketokarbonsäuren III werden mit Aminohydroxykarbonsäure-Derivaten IV (Herstellung von IV siehe S.L.Harbenson et al., J.Med.Chem. 1994, 37,2918-29) unter üblichen Peptid-Kupplungs-Methoden (siehe oben, Houben-Weyl) umgesetzt, wobei Amide VII anfallen. Diese Alkohol-Derivate VII können zu den erfindungsgemäßen Ketokarbonsäure-Derivaten I oxidiert werden. Dafür kann man verschiedene übliche Oxidationsreaktionen (siehe C.R.Larock, Comprehensive Organic Transformations, VCH Publisher, 1989, Seite 604 f.) wie zum Beispiel Swern- und Swern-analoge Oxidationen, bevorzugt mit Dimethylsulfoxid/Pyridin-Schwefeltrioxid in Lösungsmitteln wie Methylenchorid oder Tetrahydrofuran, gegebenenfalls unter Zusatz von Dimethylsulfoxid, bei Raumtemperatur oder Temperaturen von -50 bis 25°C, (T.T.Tidwell, Synthesis 1990, 857-70) oder Natriumhypochlorid/TEMPO (S.L.Harbenson et al., siehe oben), benutzen.

Wenn die Verbindungen VII α-Hydroxyester darstellen (X = O-Alkyl), können diese zu Karbonsäuren VIII hydrolysiert werden, wobei analog zu den obigen Methoden gearbeitet wird, bevorzugt aber mit Lithiumhydroxid in Wasser/Tetrahydrofuran-Gemischen bei Raumtemperatur. Die Herstellung von anderen Estern oder Amiden X erfolgt durch Umsetzung mit Alkoholen oder Aminen unter bereits beschriebenen Kupplungsbedingungen. Das Alkohol-Derivat X kann erneut zu erfindungsgemäßen Ketokarbonsäure-Derivaten I oxidiert werden.

Die Synthese der Karbonsäureester II ist teilweise bereits beschrieben worden oder entsprechend üblichen chemischen Methoden durchführbar.

Verbindungen, bei denen X eine Bindung darstellt, werden durch übliche aromatische Kupplung, zum Beispiel die Suzuki-Kupplung mit Borsäure-Derivaten und Halogeniden unter Palladiumkatalyse oder die kupferkatalytische Kupplung von aromatischen Halogeniden, hergestellt. Die Alkyl-überbrückten Reste (X= -(CH₂)ₘ-) können durch Reduktion der analogen Ketone oder durch Alkylierung der Organolithium-, z.B. ortho-Phenyloxazolidine, oder anderer Organometall-Verbindungen hergestellt werden (vgl. I.M.Dordor, et al., J.Chem.Soc. Perkin Trans. I, 1984, 1247-52).

Ether-überbrückte Derivate werden durch Alkylierung der entsprechenden Alkohole oder Phenole mit Halogeniden hergestellt.

Die Sulfoxide und Sulfone sind durch Oxidation der entsprechenden Thioether zugänglich.

Alken- und Alkin- überbrückte Verbindungen werden zum Beispiel mit Hilfe der Heck-Reaktion aus aromatischen Halogeniden und entsprechenden Alkenen und Alkinen hergestellt (vgl. I.Sakamoto et al., Chem.Pharm.Bull., 1986, 34, 2754-59).

Die Chalkone entstehen durch Kondensation aus Acetophenonen mit Aldehyden und können gegebenenfalls durch Hydrierung in die analogen Alkyl-Derivate überführt werden.

Amide und Sulfonamide werden analog den oben beschriebenen Methoden aus den Aminen und Säure-Derivaten hergestellt.

Die erfindungsgemäßen Ketobenzamide I stellen Inhibitoren von Cystein-Proteasen dar, insbesondere von Cystein-Proteasen wie die Calpaine I und die II und Cathepsine B bzw. L.

Die inhibitorische Wirkung der Ketobenzamide I wurde mit in der Literatur üblichen Enzymtests ermittelt, wobei als Wirkmaßstab eine Konzentration des Inhibitors ermittelt wurde, bei der 50% der Enzymaktivität gehemmt wird. Die Benzamide I wurden in dieser Weise auf ihre Hemmwirkung von Calpain I, Calpain II und Cathepsin B gemessen.

### Cathepsin B-Test

Die Cathepsin B-Hemmung wurde analog einer Methode von S.Hasnain et al., J.Biol.Chem. 1993, 268, 235-40 bestimmt.

Zu 88µL Cathepsin B (Cathepsin B aus menschlicher Leber (Calbiochem), verdünnt auf 5 Units in 500µM Puffer) werden 2µL einer Inhibitor-Lösung, hergestellt aus Inhibitor und DMSO (Endkonzentrationen: 100µM bis 0,01µM) gegeben. Dieser Ansatz wird 60 Minuten bei Raumtemperatur (25°C) vorinkubiert und anschließend die Reaktion durch Zugabe von 10µL 10mM Z-Arg-Arg-pNA (in Puffer mit 10% DMSO) gestartet. Die Reaktion wird 30 Minuten bei 405nm im Mikrotiterplattenreader verfolgt. Aus den maximalen Steigungen werden anschließend die IC₅₀'s bestimmt.

### Calpain I und II Test

Die Testung der inhibitorischen Eigenschaften von Calpain-Inhibitoren erfolgt in Puffer mit 50 mM Tris-HCl, pH 7,5; 0,1 M NaCl; 1 mM Dithiotreithol: 0,11 mM CaCl₂, wobei das fluorogene Calpainsubstrat Suc-Leu-Tyr-AMC (25 mM gelöst in DMSO, Bachem/Schweiz) verwendet wird (Sasaki et al. J. Biol. Chem. 1984, Vol. 259, 12489-12494). Humanes µ-Calpain wird aus Erythrozyten in Anlehnung an die Methoden von Croall und DeMartino (BBA 1984, Vol. 788, 348-355) und Graybill et al. (Bioorg. & Med. Lett. 1995, Vol. 5, 387-392) isoliert. Nach mehreren chromatographischen Schritten (DEAE-Sepharose, Phenyl-Sepharose, Superdex 200 und Blue-Sepharose) erhält man das Enzym mit einer Reinheit < 95 %, beurteilt nach SDS-PAGE, Western Blot Analyse und N-terminaler Sequenzierung. Die Fluoreszenz des Spaltproduktes 7-Amino-4-methylcoumarin (AMC) wird in einem Spex-Fluorolog Fluorimeter bei λₑₓ = 380 nm und λₑₘ = 460 nm verfolgt. In einem Meßbereich von 60 min ist die Spaltung des Substrats linear und die autokatalytische Aktivität von Calpain gering, wenn die Versuche bei Temperaturen von 12°C durchgeführt werden (siehe Chatterjee et al. 1996, Bioorg. & Med. Chem. Lett., Vol 6, 1619-1622). Die Inhibitoren und das Calpainsubstrat werden in den Versuchsansatz als DMSO-Lösungen gegeben, wobei DMSO in der Endkonzentration 2 % nicht überschreiten soll.

In einem typischen Versuchsansatz werden 10 µl Substrat (250 µm final) und anschließend 10 µl an µ-Calpain (2 µg/ml final, d.h. 18 nM) in eine 1 ml Küvette gegeben, die Puffer enthält. Die Calpain-vermittelte Spaltung des Substrats wird für 15 bis 20 min gemessen. Anschließend erfolgt die Zugabe von 10 µl Inhibitor (50 oder 100 µM Lösung DMSO) und die Messung der Inhibition der Spaltung für weitere 40 min. Kᵢ-Werte werden nach der üblichen Gleichung für reversible Hemmung bestimmt, d.h. K: = 1(vₒ/v)-1; wobei I = Inhibitorkonzentration, vₒ = Anfangsgeschwindigkeit vor Zugabe des Inhibitors; vᵢ = Reaktionsgeschwindigkeit im Gleichgewicht bedeutet.

Für das (S)-N(1-Ethoxycarbonyl-l-oxo-3-phenyl-propan-2-yl)-2-phenyl-benzamid (Beispiel 24) wurde eine K_{I} von < 10µM ermittelt. Damit ist dieses Derivat deutlich wirksamer als das sehr nahe verwandte N(1-Ethoxycarbonyl-l-oxo-3-phenyl-propan-2-yl)-benzamid (aus M.R.Angelastro et al., J.Med.Chem. 1990, 33, 11-13).

Calpain ist eine intrazelluläre Cysteinprotease. Calpain-Inhibitoren müssen die Zellmembran passieren, um den Abbau von intrazellulären Proteinen durch Calpain zu verhindern. Einige bekannte Calpain-Inhibitoren, wie zum Beispiel E 64 und Leupeptin, überwinden die Zellmembranen nur schlecht und zeigen dementsprechend, obwohl sie gute Calpain-Inhibitoren darstellen, nur schlechte Wirkung an Zellen. Ziel ist es, Verbindungen mit besserer Membrangängigkeit zu finden. Als Nachweis der Membrangängigkeit von Calpain-Inhibitoren wurden humane Plättchen verwendet.

Calpain-vermittelter Abbau der Tyrosinkinase pp60src in Plättchen Nach der Aktivierung von Plättchen wurde die Tyrosinkinase pp60src durch Calpain gespalten. Dies wurde von Oda et al. in J. Biol. Chem., 1993, Vol 268, 12603-12608 eingehend untersucht. Hierbei wurde gezeigt, daß die Spaltung von pp60src durch Calpeptin, einen Inhibitor für Calpain, verhindert werden kann. In Anlehnung an diese Publikation wurde die zellulare Effektivität der neuen Substanzen getestet. Frisches humanes, mit Zitrat versetztes Blut wurde 15 min bei 200 g zentrifugiert. Das Plättchen-reiche Plasma wurde gepoolt und mit Plättchenpuffer 1:1 verdünnt (Plättchenpuffer: 68 mM NaCl, 2,7 mM KCl, 0,5 mM MgCl₂ x 6 H₂O, 0,24 mM NaH₂PO₄ x H₂O, 12 mM NaHCO₃, 5,6 mM Glukose, 1 mM EDTA, pH 7,4). Nach einem Zentrifugations- und Waschschritt mit Plättchenpuffer wurden die Plättchen auf 10⁷ Zellen/ml eingestellt. Die Isolierung der humanen Plättchen erfolgte bei RT.

Im Testansatz wurden isolierte Plättchen (2 x 10⁶) mit unterschiedlichen Konzentrationen an Inhibitoren (gelöst in DMSO) 5 min bei 37°C vorinkubiert. Anschließend erfolgte die Aktivierung der Plättchen mit 1 µM Ionophor A23187 und 5 mM CaCl₂. Nach 5 min Inkubation wurden die Plättchen kurz bei 13000 rpm zentrifugiert und das Pellet in SDS-Probenpuffer aufgenommen (SDS-Probenpuffer: 20 mM Tris-HCl. 5 mM EDTA, 5 mM EGTA, 1 mM DTT, 0,5 mM PMSF, 5 µg/ml Leupeptin, 10 µm Pepstatin, 10 % Glycerin und 1 % SDS). Die Proteine wurden in einem 12 %igen Gel aufgetrennt und pp60src und dessen 52-kDa und 47-kDa Spaltprodukte durch Western-Blotting identifiziert. Der verwendete polyklonale Kaninchen-Antikörper Anti-Cys-src (pp60^{c-src}) war von der Firma Biomol Feinchemikalien (Hamburg) erworben worden. Dieser primäre Antikörper wurde mit einem HRP-gekoppelten zweiten Antikörper aus der Ziege (Boehringer Mannheim, FRG) nachgewiesen. Die Durchführung des Western-Blotting erfolgte nach bekannten Methoden.

Die Quantifizierung der Spaltung von pp60src erfolgte densitometrisch, wobei als Kontrollen nicht-aktivierte (Kontrolle 1: keine Spaltung) und mit Ionophor- und Kalzium-behandelte Plättchen (Kontrolle 2: entspricht 100 % Spaltung) verwendet wurden. Der ED₅₀-Wert entspricht der Konzentration an Inhibitor bei der die Intensität der Farbreaktion der 60-kDa Bande dem wert Intensität der Kontrolle 1 plus Kontrolle 2 geteilt durch 2 entspricht.

### Glutamat induzierter Zelltod an corticalen Neuronen

Der Test wurde, wie bei Choi D. W., Maulucci-Gedde M. A. and Kriegstein A. R., "Glutamate neurotoxicity in cortical cell culture". J. Neurosci. 1989,7, 357-368, durchgeführt.

Aus 15 Tage alten Mäuseembryos werden die Cortexhälften präpariert und die Einzelzellen enzymatisch (Trypsin) gewonnen. Diese Zellen (Glia und corticale Neuronen) werden in 24 Well-Platten ausgesät. Nach drei Tagen (Laminin beschichteten Platten) oder sieben Tagen (Ornithin beschichteten Platten) wird mit FDU (5-Fluor-2-desoxyuridine) die Mitosebehandlung durchgeführt. 15 Tage nach der Zellpräparation wird durch Zugabe von Glutamat (15 Minuten) der Zelltod ausgelöst. Nach der Glutamatentfernung werden die Calpaininhibitoren zugegeben. 24 Stunden später wird durch die Bestimmung der Lactatdehydrogenase (LDH) im Zellkulturüberstand die Zellschädigung ermittelt.

Man postuliert, daß Calpain auch eine Rolle im apoptotischen Zelltod spielt (M.K.T.Squier et al. J.Cell.Physiol. 1994, 159, 229-237; T.Patel et al. Faseb Journal 1996, 590, 587-597 ). Deshalb wurde in einem weiteren Modell in einer humanen Zellinie der Zelltod mit Kalzium in Gegenwart eines Kalziumionophors ausgelöst. Calpain-Inhibitoren müssen in die Zelle gelangen und dort Calpain hemmen, um den ausgelösten Zelltod zu verhindern.

### Kalzium-vermittelter Zelltod in NT2 Zellen

In der humanen Zellinie NT2 läßt sich durch Kalzium in Gegenwart des Ionophors A 23187 der Zelltod auslösen. 10⁵ Zellen/well werden in Mikrotiterplatten 20 Stunden vor dem Versuch ausplattiert. Nach diesem Zeitraum werden die Zellen mit verschiedenen Konzentrationen an Inhibitoren in Gegenwart von 2,5 µM Ionophor und 5 mM Kalzium inkubiert. Dem Reaktionsansatz werden nach 5 Stunden 0,05 ml XTT (Cell Proliferation Kit II, Boehringer Mannnheim ) hinzugegeben. Die optische Dichte wird ungefähr 17 Stunden später, entsprechend den Angaben des Herstellers, in dem EASY READER EAR 400 der Firma SLT bestimmt. Die optische Dichte, bei der die Hälfte der Zellen abgestorben sind, errechnet sich aus den beiden Messungen ohne Inhibitoren, die in Abwesenheit und Gegenwart von Ionophor inkubiert wurden. Die Konzentration des Inhibitors, die diese halb-maximale optische Dichte erreicht, stellt den IC₅₀-Wert dar.

Bei einer Reihe von neurologischen Krankheiten oder psychischen Störungen tritt erhöhte Glutamat-Aktivität auf, die zu Zuständen von Übererregungen oder toxischen Effekten im zentralen Nervensystem (ZNS) führt.

Substanzen, die die durch Glutamat vermittelten Effekte hemmen, können somit zur Behandlung dieser Krankheiten eingesetzt werden. Glutamat-Antagonisten, dazu gehören insbesondere auch NMDA-Antagonisten bzw. deren Modulatoren und die AMPA-Antagonisten, eignen sich zur therapeutischen Anwendung als Mittel gegen neurodegenerative Krankheiten (Chorea Huntington und Parkinsonsche Krankheiten), neurotoxische Störungen nach Hypoxie, Anoxie oder Ischämie, wie sie nach "Stroke" auftreten, oder auch als Antiepileptika, Antidepressiva und Anxiolytika (vgl. Arzneim. Forschung 1990, 40, 511 - 514; TIPS, 1990, 11, 334 - 338 und Drugs of the Future 1989, 14 (11), 1059 - 1071).

Durch intrazerebrale Applikation von exzitatorischen Aminosäuren (= EAA = Excitatory Amino Acids) wird eine so massive Übererregung induziert, daß diese in kurzer Zeit zu Krämpfen und zum Tod der Tiere führt. Durch systemische - z.B. intraperitoneale - Gabe von zentral-wirksamen EAA-Antagonisten lassen sich diese Symptome hemmen. Da die exzessive Aktivierung von EAA-Rezeptoren des Zentralnervensystems in der Pathogenese verschiedener neurologischer Erkrankungen eine bedeutende Rolle spielt, kann aus dem nachgewiesenen EAA-Antagonismus in vivo auf die therapeutische Verwendbarkeit der Substanzen gegen derartige ZNS-Erkrankungen geschlossen werden. Hierzu zählen u.a. fokale und globale Ischämien, Trauma, Epilepsien sowie verschiedene neurodegenerative Erkrankungen, wie Chorea Huntington, Parkinson Krankheit u.a.

Es wurde bereits gezeigt, daß auch Calpain-Inhibitoren in Zellkulturen protektive Wirkung gegen den durch EAA ausgelösten Zelltod zeigen (H. Cauer et al., Brain Research 1993, 607, 354-356; Yu Cheg und A.Y. Sun, Neurochem. Res. 1994, 19, 1557-1564). Die in dieser Anmeldung enthaltenen Calpain-Inhibitoren sind überraschenderweise sogar gegen die durch EAA (z.B. NMDA oder AMPA) ausgelösten Krämpfe wirksam und zeigen damit auf eine therapeutische Verwendung in den oben genannten ZNS-Erkrankungen hin.

Die Ketobenzamide I stellen Inhibitoren von Cystein-Derivaten wie Calpain I bzw. II und Cathepsin B bzw. L dar und können somit zur Bekämpfung von Krankheiten, die mit einer erhöhten Enzymaktivität der Calpain-Enzyme oder Cathepsin-Enzyme verbunden sind, dienen. Sie eignen sich daher zur Behandlung von neurodegenerativen Krankheiten, die nach Ischämie, Trauma, Subarachnoidal-Blutungen und Stroke auftreten und zu denen insbesondere Hirnschlag und Schädeltrauma zählen, und von neurodegenerativen Krankheiten wie multipler Infarkt-Dementia, Alzheimer Krankheit und Huntington Krankheit und weiterhin zur Behandlung von Schädigungen des Herzens nach cardialen. Ischämien, Schädigungen der Nieren nach renalen Ischämien, Skelettmuskelschädigungen, Muskeldystrophien, Schädigungen, die durch Proliferation der glatten Muskelzellen entstehen, coronaren Vasospasmen, cerebralen Vasospasmen, Katarakten der Augen, Restenosis der Blutbahnen nach Angioplastie.

Zudem können die Benzamide I zur Chemotherapie von Tumoren und deren Metastasen nützlich sein und zur Behandlung von Krankheiten, bei denen ein erhöhter Interleukin-1-Spiegel auftritt, wie bei Entzündungen und rheumatischen Erkrankungen, dienen. Die erfindungsgemäßen Arzneimittelzubereitungen enthalten neben den üblichen Arneimittelhilfstoffen eine therapeutisch wirksame Menge der Verbindungen I.

Für die lokale äußere Anwendung, zum Beispiel in Puder, Salben oder Sprays, können die Wirkstoffe in den üblichen Konzentrationen enthalten sein. In der Regel sind die Wirkstoffe in einer Menge von 0,001 bis 1 Gew.-%, vorzugsweise 0,01 bis 0,1 Gew.-% enthalten.

Bei der inneren Anwendung werden die Präperationen in Einzeldosen verabreicht. In einer Einzeldosis werden pro kg Körpergewicht 0,1 bis 100 mg gegeben. Die Zubereitung können täglich in einer oder mehreren Dosierungen je nach Art und Schwere der Erkrankungen verabreicht werden.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes hydriertes Ricinusöl, Polyacrylsäure, Polyethylenglykol, Polyethylenglykostearat, ethoxylierte Fettalkohole, Paraffinöl, Vaseline und Wollfett, verwendet werden. Für die innere Anwendung eignen sich zum Beispiel Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitungen verwendeten Stoffe sind toxikologisch unbedenklich und mit dem jeweiligen Wirkstoff verträglich. Die Herstellung der Arzneimittelzubereitungen erfolgt in üblicher Weise, zum Beispiel durch Vermischung des Wirkstoffes mit anderen üblichen Trägerstoffen und Verdünnungsmitteln.

Die Arzeinimittelzubereitungen können in verschiedenen Applikationsweisen verbreicht werden, zum Beispiel peroral, parenteral wie intravenös durch Infusion, subkutan, intraperitoneal und topisch.

So sind Zubereitungsformen wie Tabletten, Emulsionen, Infusionsund Injektionslösungen, Pasten, Salben, Gele, Cremes, Lotionen, Puder und Sprays möglich.

### Beispiele

### Beispiel 1

### (S)-2(E-2-(Naphth-2-yl)-ethen-1-yl)-N(1-(N(3-morpholino-1-yl-propan-1-yl)carbamoyl-1-oxo-3-phenyl-propan-2-yl)-benzamid

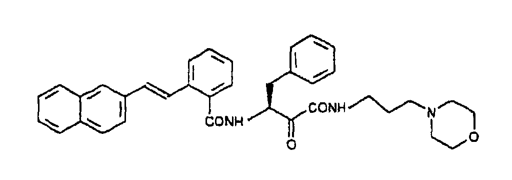

a) 2-(2-(E-Naphth-2-yl)-ethen-1-yl)-benzoesäureethylester
   29.7g (0.13Mol) 2-Vinylnaphthalin, 25g (0.16Mol) 2-Brombenzoesäureethylester, 22.5ml (0.16Mol) Triethylamin, 0.54g Palladiumdiacetat und 1.44g Triphenylphosphin wurden in 200ml Acetonitril für 20 h auf 100°C erhitzt. Danach wurde alles auf Wasser gegossen und mehrmals mit Essigsäuereethylester extrahiert. Die organische Phase wurde im Vakuum eingeengt und der Rückstand chromatographisch an Kieselgel gereinigt. Ausbeute: 34g (71%).
b) 2-(E-2-(Naphth-2-yl)-ethen-1-yl)-benzoesäure
   34g (112.5mMol) des Zwischenproduktes 1a wurden in 200ml Tetrahydrofuran gelöst und mit 9.5g (168,7mMol) 80%igem Kaliumhydroxid, gelöst in 150ml Wasser, versetzt. Alles wurde 10h unter Rückfluß erhitzt. Anschließend wurde das Reaktionsgemisch mit konzentrierter Salzsäure acidifiziert und mit Essigsäureethylester extrahiert. Die organische Phase wurde mit Wasser gewaschen, getrocknet und im Vakuum eingeengt. Der Rückstand wurde noch mit wenig Essigester behandelt und abgesaugt. Ausbeute 23.8g (78%).
c) (S)-O(tert.-Butyl)-N(1-(N(3-morpholino-1-yl-propan-1-yl)carbamoyl-3-phenyl-propan-1-ol-2-yl)-carbamat
   Zu 2.95g (10mMol) O(tert.-Butyl)-2-(S)-N(1-carboxy-2-hydroxy-3-phenyl-propan-1-ol-2-yl)-carbamat (S.L. Harbeson et al., J.Med.Chem. 1994, 37, 2918-29) und 1.4g (10mMol) N-(3-Aminopropan-1-yl)morpholin in 50ml wasserfreiem Dimethylformamid wurden bei -5°C nacheinander 1.6g (10mMol) Cyanphosphorsäurediethylester und 1.0g (10mMol) Triethylamin zugegeben. Alles wurde 1h bei -5°C und danach 16h bei Raumtemperatur gerührt. Anschließend wurde alles auf Wasser gegeben und mit Essigester extrahiert. Die organische Phase wurde mit wäßriger Zitronensäure-Lösung extrahiert. Diese wäßrige Phase wurde danach mit verdünnter Natronlauge alkalisiert und mit Essigester extrahiert. Die organische Phase wurde getrocknet und im Vakuum eingeengt, wobei man 2.3g (55%) Produkt erhielt.
d) 3(3)-3-Amino-2-hydroxy-3-phenyl-N(-3-morpholin-1-yl-propan-1yl)-buttersäureamid
   2.1g (5mMol) des Zwischenproduktes 1c wurden in 60ml Methylenchlorid gelöst und mit 60ml Trifluoressigsäure versetzt. Es wurde 30 min bei Raumtemperatur gerührt. Danach wurde der Ansatz im Vakuum eingeengt und der Rückstand aus Methylenchorid/Ether umgefällt. Man erhielt 2.4g Rohprodukt.
e) 2-(S)-2(E-2-(Naphth-2-yl)-ethen-1-y1)-N(1-(N(3-morpholino-1-yl-propan-l-yl)carbamoyl)-1-hydroxy-3-phenyl-propan-2-yl)-benzamid
   Zu 2.4g (4mMol) des Zwischenproduktes 1d und 1.1g (4mMol) des Zwischenproduktes 1b in 30ml wasserfreiem Dimethylformamid wurden bei -5°C nacheinander 0.65g (4mMol) Cyanphosphorsäurediethylester und 0.8g (8mMol) Triethylamin gegeben. Anschließend wurde alles 1h bei -5°C und weitere 16h bei Raumtemperatur gerührt. Danach gab man 200ml Wasser zu und extrahierte mit Diethylether. Die wäßrige Phase wurde mit verdünnter Natronlauge neutralisiert und danach mit Essigsäureethylester extrahiert. Diese organische Phase wurde getrocknet und im Vakuum eingegengt. Der Rückstand wurde aus Essigsäureethylester umkristallisiert. Ausbeute: 0.8g (35%).
f) 2-(S)-2(E-2-(Naphth-2-yl)-ethen-1-yl)-N(1-(N(3-morpholino-1-y 1-propan-1-yl)carbamoyl)-1-oxo-3-phenyl-propan-2-yl)-benzamid
   Zu 0.46g (0.8mMol) des Zwischenproduktes 1e und 0.3g (3.2mMol) Triethylamin in 8ml Dimethylsulfoxid wurden bei Raumtemeperatur 0.38g (2.4mMol) Pyridin-Schwefeltrioxid-Komplex, gelöst in 4 ml Dimethylsulfoxid, gegeben. Alles wurde 16h gerührt. Danach wurde der Ansatz zuerst mit Wasser verdünnt und danach mit Methylenchlorid extrahiert. Die organische Phase wurde getrocknet und im Vakuum eingeengt. Der Rückstand wurde mit Ether behandelt, wobei 0.3g (65%) Produkt anfielen.
   1H-NMR (CDCl₃) : δ = 1.7(2H), 2.4(6H), 3.2(1H), 3.5(3H), 3.7(4H), 5.8(1H), 6.5(1H), 7.0-8.0(19H) und 8.8(1H) ppm.

### Beispiel 2

### (S)-2(E-2-Naphth-2-yl)-ethen-1-yl)-N(1-carbamoyl-1-oxo-3-phenylpropan-2-yl)-benzamid

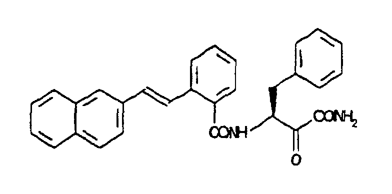

a) 2(S)-O(tert.-Butyl)-N(1-carbamoyl-3-phenyl-propan-1-ol-2-yl)-carbamat
   217.7g (60mMol) O(tert.-Butyl)-2(S)-N(1-carboxy-2-hydroxy-3-phenyl-propan-1-ol-2-yl)-carbamat (S.L. Harbeson et al., J.Med.Chem. 1994, 37, 2918-29) wurden analog Beispiel 1c mit ethanolischer Ammoniak-Lösung umgesetzt. Ausbeute: 13.5g (76%).
b) 3-(S)-3-Amino-2-hydroxy-3-phenyl-buttersäureamid
   13.4g (45.5mMol) der Zwischenverbindung 2a wurden analog Beispiel 1d umgesetzt. Man erhielt 12.3g (88%) Produkt.
c) 2-(S)-2(E-2-(Naphth-2-yl)-ethen-1-yl)-N(1-carbamoyl-1-hydroxy -3-phenyl-prop-2-yl)-benzamid
   Zu 1.65g (6mMol) der Zwischenverbindung 2b und 0.81g (6mMol) 1-Hydroxybenzotriazol (HOBT) in 10ml wasserfreiem Dimethylformamid wurden bei -5°C nacheinander 1.26g (6.6mMol) N'-(3-Dimethylaminopropyl)-N-ethylcarbodiimidhydrochlorid (EDC), 1.85g (6mMol) der Zwischenverbindung 1b und 1.2g (12mMol) N-Methylmorpholin zugegeben. Danach wurde alles 1h bei -5°C und noch weitere 16h bei Raumtemperatur gerührt. Anschließend gab man Wasser hinzu und saugte den Niederschlag ab. Ausbeute: 1.3g (48%) Produkt.
d) (S)-2(2- (Naphth-2-yl)-ethen-1-yl) -N(1-carbamoyl-1-oxo-3-phenyl-propan-2-yl)-benzamid
   0.45g (1mMol) der Zwischenverbindung 2c wurden analog Beispiel 1f oxidiert. Ausbeute: 0.28g (62%).
   MS : m/e = 458(M⁺).

### Beispiel 3

### 2-(S)-2(E-2-(3,4-Dimethoxyphenyl)-ethen-1-yl)-N(1-carbamoyl-1-oxo-3-phenyl-propan-2-yl)-benzamid

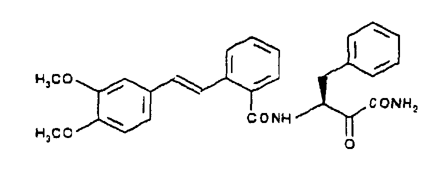

a) 2-(E-2-(3,4-Dimethoxyphenyl)-ethen-1-yl)-benzoesäureethylester
   5g (30.5mMol) 3,4-Dimethoxystyrol wurden analog Beispiel 1a mit 2-Brombenzoesäureethylester in Dimethylformamid bei 120°C umgesetzt. Man erhielt 7.2g (94%) Produkt.
b) 2-(E-2-(3,4-Dimethoxyphenyl)-ethen-1-yl)-benzoesäure
   7g (22mMol) des Zwischenproduktes 3a wurden analog Beispiel 1b mit 4M Natronlauge verseift. Ausbeute: 6.2g (98%).
c) 2-(S)-2(2-(3,4-Dimethoxyphenyl)-ethen-l-yl)-N(1-carbamoyl-1-hydroxy-3-phenyl-propan-2-yl)-benzamid
   1.7g (6mMol) der Zwischenverbindung 2b wurden analog Beispiel 2c mit der Verbindung 3b umgesetzt. Ausbeute: 2.1g (76%).
d) 2-(S)-2(E-2-(3,4-Dimethoxyphenyl)-ethen-1-yl)-N(1-carbamoyll-oxo-3-phenyl-propan-2-yl)-benzamid
   0.45g (1mMol) der Zwischenverbindung 3c wurden analog Beispiel 1f oxidiert. Man erhielt 0.28g (62%) Produkt. MS: m/e = 479(M⁺).

### Beispiel 4

### (S)-4(2-Naphthylamido)methyl-N(1-carbamoyl-1-oxo-3-phenyl-propan-2-yl)-benzamid

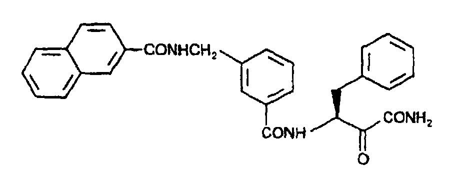

a) 4-(2-Naphthylamido)methyl-benzoesäure
   Zu 10g (66.2mMol) 4-Aminomethyl-benzoesäure in 150ml Pyridin wurden bei 10°C 12.6g (66.2mMol) 2-Naphthoesäurechlorid, gelöst in 150ml Tetrahydrofuran, getropft. Alles wurde anschließend 16h bei Raumtemperatur gerührt. Der Ansatz wurde dann im Vakuum eingeengt und der anfallende Rückstand chromatographisch (Fließmittel: Methylenchlorid/Methanol = 10/1) gereinigt, wobei 11.3g (56%) Produkt anfielen.
b) 4 (2-Naphthylamido)methyl-N (-3(S)-1-carbamoyl-1-hydroxy-3-phenyl-propan-2-yl)-benzamid
   1.2g (4mMol) des Zwischenproduktes 4a wurden analog Beispiel 2c mit 3(S)-3-Amino-2-hydroxy-3-phenyl-buttersäureamid 2b umgesetzt, wobei 1.7g (88%) Produkt anfielen.
c) (S)-4(2-Naphthylamido)methyl-N(1-carbamoyl-1-oxo-3-phenylpropan-2-yl)-benzamid
   0.48g (1mMol) der Zwischenverbindung 4b wurden analog Beispiel 1f oxidiert. Ausbeute: 0.31g (65%).
   1H-NMR (D₆-DMSO): δ = 2.9(1H), 3.2(1H), 4.5(2H), 5.2(1H), 7.0-8.0(16H), 8.2(1H), 8.7(1H) und 9.1(2H) ppm.

### Beispiel 5

### (S)-2-phenyl-N(1-carbamoyl-1-oxo-3-phenyl-propan-2-yl)-benzamid

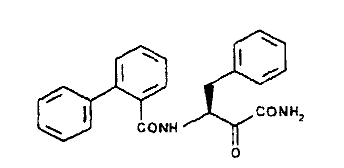

a) 2-Phenyl-N(-3(S)-1-carbamoyl-1-hydroxy-3-phenyl-propan-2-yl)-benzamid
   0.8g (4mMol) Biphenyl-2-carbonsäure und 1.2g (4mMol) der Zwischenverbindung 2b wurden analog Beispiel 2c umgesetzt.
   Ausbeute: 1.2g (80%).
b) (S)-2-Phenyl-N(1-carbamoyl-1-oxo-3-phenyl-propan-2-yl)-benzamid
   0.75g(2mMol) der Zwischenverbindung 5a wurden analog Beispiel 1f oxidiert. Ausbeute: 0.35g (47%).
   1H-NMR (D₆-DMSO) : δ = 2.8(1H), 3.1(1H), 5.2(1H), 7.0-7.5(14H), 7.9(1H), 8.1(1H) und 8.9(1H) ppm.

### Beispiel 6

### (S)-2(Naphth-2-ylmethyl) -N(1-carbamoyl-l-oxo-3-phenyl-propan-2-yl)-benzamid

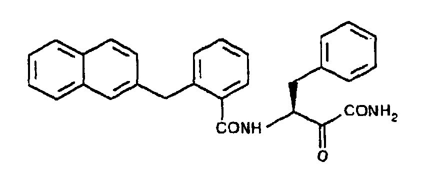

a) 4,4-Dimethyl-2-(2-(naphth-2-yl-hydroxymethyl)phenyl)-2-oxazolin
   Zu 25g (0.14Mol) 4,4-Dimethyl-2-phenyl-2-oxazolin und 0.1g Triphenylmethan in 400ml wasserfreiem Tetrahydrofuran wurden bei -78°C langsam 104ml einer 1.6M Butyllithium-Lösung zugetropft. Alles wurde für 1h gerührt. Danach ließ man auf -30° erwärmen und tropfte eine Lösung aus 20.3g (0.13Mol) 2-Naphthaldehyd, gelöst in 200ml wasserfreiem Tetrahydrofuran, zu. Es wurde noch 1h bei -20 bis -30°C gerührt. Anschließend ließ man die Reaktionslösung auf Raumtemperatur erwärmen und entfernte das Lösungsmittel im Vakuum. Der Rückstand wurde in Eiswasser gegeben, das anschließend mit Ether extrahiert wurde. Die organische Phase wurde getrocknet und im Vakuum eingeengt. Der Rückstand wurde chromatographisch gereinigt (Fließmittel: n-Heptan/Aceton = 40/3). Ausbeute: 25.3g (54%).
b) 3-Napth-2-yl-phthalid
   22g (66mMol) des Zwischenproduktes 6a wurden in einem Gemisch aus 250ml Ethanol und 100ml 1M Salzsäure 2h unter Rückfluß gekocht. Danach wurde das Ethanol im Vakuum entfernt und der entstandene Niederschlag abgesaugt. Ausbeute: 16.4g (95%).
c) 2-Naphth-2-yl-benzoesäure
   16g (61.5mMol) des Zwischenproduktes 6b wurden in einem Gemisch aus 100ml Tetrahydrofuran und 250ml Ethanol gelöst und, nachdem man 5g Palladium/Bariumsulfat zugegeben hatte, hydriert. Danach wurde filtriert und das Filtrat im Vakuum eingeengt. Der Rückstand wurde in Toluol umkristallisiert, wobei 13.6g (85%) Produkt anfielen.
d) 2(Naphth-2-yl)methyl-N(-3(S)-1-carbamoyl-1-hydroxy-3-phenylpropan-2-yl)-benzamid
   1.05g (4mMol) des Zwischenproduktes 6c wurden analog Beispiel 2c mit dem Zwischenprodukt 2b umgesetzt, wobei 1.7g (97%) des Produktes anfielen.
e) (S)-2(Naphth-2-yl)methyl-N(1-carbamoyl-1-oxo-3-phenyl-propan-2-yl)-benzamid
   0.88g (2mMol) der Zwischenverbindung 6d wurden analog Beispiel 1f oxidiert. Ausbeute: 0.52g (60%).
   1H-NMR (D₆-DMSO): δ = 2.8(1H), 3.2(1H), 4.1(2H), 5.3(1H), 7.1-8.0(17H), 8.1(1H) und 8.9(1H) ppm.

### Beispiel 7

### (S)-3(2-Naphthyl)sulfonamido-N(1-carbamoyl-1-oxo-3-phenyl-propan-2-yl)-benzamid

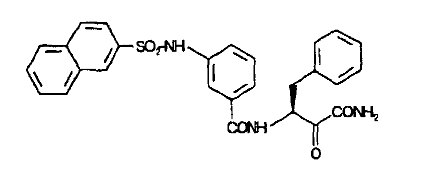

a) 3(2-Naphthylsulfonamido)-benzoesäureethylester
   Zu 25g (0.15 Mol) 3-Aminobenzoesäureethylester und 63ml (0.45Mol) Triethylamin in 400ml Tetrahydrofuran werden bei 0°C 34.3g (0.15 Mol) 2-Naphthalinsulfonsäurechlorid, gelöst in 250ml Tetrahydrofuran, zugetropft. Danach erwärmt man alles 1h unter Rückfluß. Das organische Lösungsmittel wurde im Vakuum entfernt und der Rückstand zwischen Essigester und Wasser verteilt. Die Essigester-Phase wurde getrocknet und im Vakuum eingeengt. Ausbeute: 55g (100%).
b) 3(2-Naphthylsulfonamido)-benzoesäure
   55g(0.15Mol) der Zwischenverbindung 7a wurden in 400ml Tetrahydrofuran gelöst und mit 400ml 4M Natronlauge versetzt. Alles wurde 1.5h bei 60°C gerührt. Das organische Lösungsmittel wurde im Vakuum entfernt. Die verbleibende wäßrige Phase wurde in verdünnter Salzsäure eingerührt. Der anfallende Niederschlag wurde in Essigester gelöst, mit Wasser gewaschen, getrocknet und im Vakuum eingeengt. Der Rückstand wurde noch mit Methylenchlorid behandelt. Danach erhielt man 37.3g (75%) Produkt.
c) 3(2-Naphthyl)sulfonamido-N(-3(S)-1-carbamoyl-1-hydroxy-3-phenyl-propan-2-yl)-benzamid
   0.55g (1.68mMol) der Zwischenverbindung 7b wurden analog Beispiel 2c mit der Verbindung 2b umgesetzt. Ausbeute: 0.72g (86%).
d) (S)-3(2-Naphthyl)sulfonamido-N(1-carbamoyl-1-oxo-3-phenylpropan-2-yl)-benzamid
   0.7g (1.4mMol) der Zwischenverbindung 7c wurden analog Beispiel 1f oxidiert. Ausbeute: 0.68g (98%).
   1H-NMR (D₆-DMSO) : δ = 2.9(1H), 3.1(1H), 5.2(1H), 7.0-8.1(17H), 8.2(1H), 8.8(1H) und 10.5(1H) ppm.

### Beispiel 8

### (S)-3(2-Naphthyl)sulfonamido-N(1-N(3-(imidazol-1-yl-propan-1-yl)carbamoyl-1-oxo-3-phenyl-propan-2-yl)-benzamid

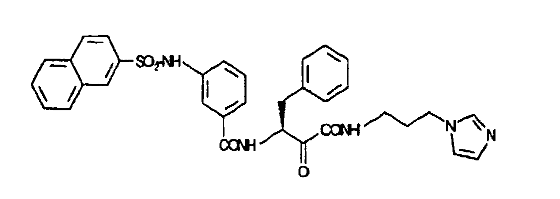

a) 3(S)-3-Amino-2-hydroxy-4-phenylbuttersäureethylester
   28g (0 12Mol) 3(S)-3-Amino-2-hydroxy-4-phenylbuttersäure (S.L. Harbeson et al., J.Med.Chem. 1994, 37, 2918-29) wurden 3h in 500ml 1M ethanolischer Chlorwasserstoff-Lösung unter Rückfluß gekocht. Danach wurde alles im Vakuum eingeengt und der Rückstand zwischen Wasser und Essigester verteilt. Die Essigester-Phase wurde mit wäßriger NatriumhydrogencarbonatLösung alkalisiert, wobei ein Öl ausfiel. Dieses Öl wurde in Essigester aufgenommen, getrocknet und im Vakuum eingeengt.
   Ausbeute: 18g.
b) 3(Naphth-2-yl)sulfonamido-N(2(S)-1-ethoxycarbonyl-1-hydroxy-3-phenyl-propan-2-yl)-benzamid
   16.5g (50.4mMol) der Zwischenverbindung 7b und 11.2g (50.4mMol) der Verbindung 8a wurden analog Beispiel 2c umgesetzt. Ausbeute: 7.8g (30%).
c) 3(2-Naphthyl)sulfonamido-N(2(S)-1-carboxy-1-hydroxy-3-phenylpropan-2-yl)-benzamid
   7.8g (14.6mMol) der Zwischenverbindung 8b wurden in 150 ml Tetrahydrofuran gelöst und mit 1.1 g (44mMol) Lithiumhydroxid, gelöst in 20ml Wasser, versetzt. Alles wurde 1h bei Raumtemperatur gerührt. Das organische Lösungsmittel wurde danach im Vakuum entfernt und die wäßrige Phase mit 1M Salzsäure schwach sauer gestellt. Der anfallende Niederschlag wurde abgesaugt. Ausbeute: 7.2g (98%).
d) 3(Naphth-2-yl)sulfonamido-N(2(S)-1-N(3-(imidazol-1)-yl-propan-1-yl)carbamoyl-l-hydroxy-3-phenyl-propan-2-yl)-benzamid 1g (2mMol) der Zwischenverbindung 8c wurden analog Beispiel 2c mit 3-Aminopropan-1-yl-1-imidazol umgesetzt. Ausbeute: 0.63g (53%).
e) (S)-3(2-Naphthyl)sulfonamido-N(1-N(3-(imidazol-1-yl-propan-1-yl)carbamoyl-1-oxo-3-phenyl-propan-2-yl)-benzamid
   0.6g (0.98mMol) der Zwischenverbindung 8d wurden analog Beispiel 1f oxidiert, wobei 0.55g (92%) Produkt anfielen.

### Beispiel 9

### (S)-N(1-N(N-Benzyl-piperidin-4-yl)-carbamoyl-1-oxo-3-phenyl-propan-2-yl)-3(naphth-2-yl)sulfonamido)-benzamid

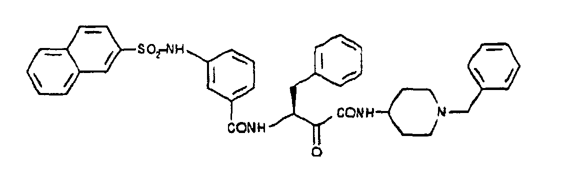

a) N(2(S)-1-N(N-Benzyl-piperidin-4-yl)-carbamoyl-1-hydroxy-3-phenyl-propan-2-yl)-3(naphth-2-yl)sulfonamido-benzamid
   1g (2mMol) der Zwischen Verbindung 8c und 4-Amino-N-benzylpiperidin wurden analog Beispiel 2c umgesetzt, wobei 0.67g (50%) Produkt anfielen.
b) (S)-N(1-N(N-Benzyl-piperidin-4-yl)carbamoyl-1-oxo-3-phenylpropan-2-yl)-3(naphth-2-yl)sulfonamido)-benzamid
   0.65g (1mMol) der Zwischenverbindung 9a wurden analog Beispiel 1f oxidiert, wobei 0.59g (91%) Produkt anfielen.

### Beispiel 10

### (S)-2(E-2-(3,4-Dimethoxyphenyl)-ethen-1-yl)-N(1-N(3-morpholino-1-yl-propan-1-yl)carbamoyl)-1-oxo-3-phenyl-propan-2-yl)-benzamid

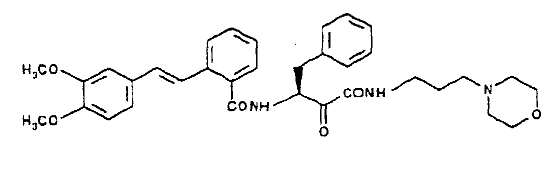

a) 2(E-2-(3,4-Dimethoxyphenyl)-ethen-1-yl)-N(2(S)-1-N(3-morpholino-1-yl-propan-1-yl)carbamoyl)-1-hydroxy-3-phenylpropan-2-yl)-benzamid
   1.7g (6mMol) der Zwischenverbindung 3b wurden analog Beispiel 2c mit der Verbindung 2b umgesetzt. Ausbeute: 1.2 g (34%).
b) (S)-2(E-2-(3,4-Dimethoxyphenyl)-ethen-1-yl)-N(1-N(3-morpholino-1-yl-propan-1-yl)carbamoyl)-1-oxo-3-phenyl-propan-2-yl)-benzamid
   0.6g (1mMol) der Zwischenverbindung wurden analog Beispiel 1f oxidiert. Ausbeute: 0.12g (20%).
   1H-NMR (CDCl₃) : δ = 1.8(2H), 2.4-2.7(6H), 3.1(1H), 3.5(2H), 3.6-3.8(5H), 3.9(6H), 5.7(1H), 6.3(1H), 6.8-7.9(14H) und 8.5(1H) ppm.

### Beispiel 11

### (S)-3(Chinolin-8-yl)sulfonamido)-N(1-carbamoyl-1-oxo-3-phenyl-propan-2-yl)-benzamid

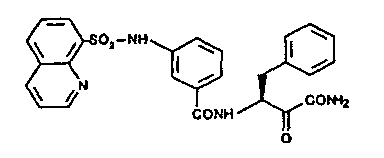

a) 8-Chinolyl-N(3-ethoxycarbonyl)-sulfonsäureamid
   5g (30.3mMol) 3-Aminobenzoesäureethylester wurden analog Beispiel 7a mit 8-Chinolinsulfonsäurechlorid bei 0°C umgesetzt, wobei 5.9g (76%) Produkt anfielen.
b) N(3-Carboxy)-8-chinolyl-sulfonsäureamid
   5.9g der Zwischenverbindung 11a wurden analog Beispiel 1b verseift. Ausbeute: 5.1g (95%).
c) 3 (Chinolin-8-yl)sulfonamido)-N(-3(S)-1-carbamoyl-1-hydroxy-3-phenyl-propan-2-yl)-benzamid
   1g (3mMol) der Zwischenverbindung 2b wurden mit 0.95g (3mMol) derVerbindung 11b analog Beispiel 2c umgesetzt, wobei 1.3g (87%) Produkt anfielen.
d) (S)-3(8-Chinolinyl)sulfonamido)-N(1-carbamoyl-1-oxo-3-phenylpropan-2-yl)-benzamid
   1.2g (2.4mMol) der Zwischenverbindung 11c wurden analog Beispiel 1f oxidiert. Ausbeute: 0.8g (70%).
   1H-NMR (D₆-DMSO) : δ = 2.9(1H), 3.1(1H), 5.2(1H), 7.0-8.8(17), 8.1(1H) und 10.2(1H) ppm.

### Beispiel 12

### (S)-4(2-Bromphenylsulfonamido)methyl-N(1-carbamoyl-1-oxo-3-phenyl-propan-2-yl)-benzamid

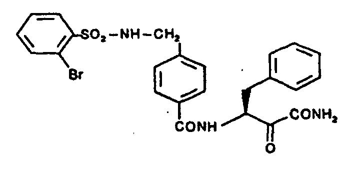

a) O-(tert.-Butyl)-N-(4-ethoxycarbonyl-benzyl)-carbamat
   7g (34.7mMol) 4-Aminomethylbenzoesäureethylester und 9.6ml (39.4mMol) Triethylamin wurden 150ml Tetrahydrofuran/Dimethylformamid (2:1) gelöst und bei 0°C mit einer Lösung aus 8g (36.5mMol) BOC-anhydrid in 100ml Tetrahydrofuran tropfenweise versetzt. Alles wurde 16h bei Raumtemperatur gerührt. Anschließend wurde der Ansatz im Vakuum eingeengt und der Rückstand zwischen Wasser und Essigester verteilt. Die organische Phase wurde getrocknet und im Vakuum eingeengt, wobei 8.5g (93%) des Produktes anfielen.
b) O-(tert.-Butyl)-N-(4-carboxybenzyl)-carbamat
   8.3g (31.3mMol) der Zwischenverbindung 12a wurden analog Beispiel 8c hydrolysiert, wobei 7.3g (93%) des Produktes anfielen.
c) 4-(tert.-Butyloxyamido)methyl-N(-3(S)-1-carbamoyl-1-hydroxy-3-phenyl-propan-2-yl)-benzamid
   7g (27.9mMol) der Zwischenverbindung 12b wurden analog Beispiel 2c mit der Verbindung 2b umgesetzt. Ausbeute: 9.2g (77%) .
d) 4-Aminomethyl-N(2(S)-1-carbamoyl-1-hydroxy-3-phenyl-propan-2-yl)-benzamid
   9.0g (21mMol) der Zwischenverbindung 12c wurden analog Beispiel 1d mit Trifluoressigsäure gespalten. Ausbeute: 10.8g (100%).
e) 4-(Bromphenylsulfonamido)methyl-N(2(S)-1-carbamoyl-1-hydroxy-3-phenyl-propan-2-yl)-benzamid
   1.5g (3.4mMol) der Zwischenverbindung 12d wurden analog Beispiel 7a mit 2-Brombenzolsulfonsäurechlorid bei 0°C umgesetzt, wobei 1.2g (69%) Produkt anfielen.
f) (S)-4(2-Bromphenylsulfonamido)methyl-N(1-carbamoyl-1-oxo-3-phenyl-propan-2-yl)-benzamid
   1.05g (1.9mMol) der Zwischenverbindung 12e wurden analog Beispiel 1f oxidiert. Ausbeute: 0.78g (75%).
   1H-NMR (D₆-DMSO) : δ = 2.9(1H), 3.2(1H), 4.2(2H), 5.3(1H), 7.0-8.0(15H), 8.4(1H) und 8.8(1H) ppm.

### Beispiel 13

### (S)-N(1-N(3-Morpholin-1-yl-3-propan-1-yl)-carbamoyl-1-oxo-3-phenyl-propan-2-yl)-2(naphth-2-ylmethyl)-benzamid

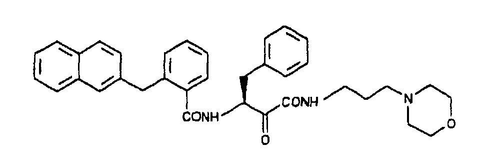

a) O-(tert.-Butyl)-N(2(S)-1(N-3-morpholin-1-yl-propan-1-yl)carbamoyl-2-hydroxy-3-phenyl-propan-2-yl)carbamat
   19.2g (65mMol) O(tert.-Butyl)-2-(S)-N(1-carboxy-2-hydroxy-3-phenyl-propan-1-ol-2-yl)-carbamat (S.L. Harbeson et al., J.Med.Chem. 1994, 37, 2918-29) wurden analog Beispiel 2c mit 1(Amino-propan-1-yl)morpholin umgesetzt, wobei 23.5g (85%) Produkt anfielen.
b) 3(S)-3-Amino-2-hydroxy-N(3-morpholin-1yl-propan-1-yl)-4-phenyl-buttersäureamid
   23.3g (55.3mMol) der Zwischenverbindung 13a wurden analog Beispiel 1d mit Trifluoressigsäure gespalten, wobei 28g eines Rohproduktes anfielen, das ungereinigt weiter umgesetzt wurde.
c) N(2(S)-1-N(3-Morpholin-1-yl-propan-1-yl)carbamoyl-1-hydroxy-3-phenyl-propan-2-yl)-2(naphth-2-ylmethyl)-benzamid
   1.57g (6mMol) der Zwischenverbindung 6c wurden analog Beispiel 2c mit der Verbindung 13b umgesetzt. Ausbeute: 1.1g (32%).
d) (S)-N(1-N(3-Morpholin-1-yl-3-propan-1-yl)-carbamoyl-1-oxo-3-phenyl-propan-2-yl)-2(naphth-2-ylmethyl)-benzamid
   0.57g (1mMol) der Zwischenverbindung 13c wurden analog Beispiel 2c oxidiert. Ausbeute: 0.14g (25%).
   1H-NMR (D₆-DMSO) : δ = 1.6(2H), 2.2(6H), 2.9(1H), 3.2(3H), 3.5(4H), 4.1(2H), 5.3(1H), 7.0-7.9(16H) und 8.9(1H) ppm.

### Beispiel 14

### (S)-N(1-N(3-Morpholin-1-yl-3-propan-1-yl)carbamoyl-1-oxo-3-phenyl-propan-2-yl)-4-(napth-2-ylamido)methyl-benzamid

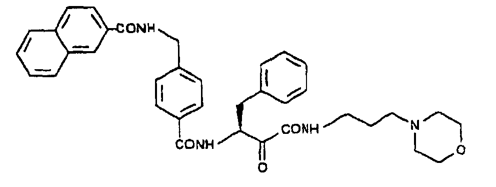

a) N(2(S)-1-N(3-Morpholin-1-yl-3-propan-1-yl)carbamoyl-1-hydroxy-3-phenyl-propan-2-yl)-4-(napth-2-ylamidomethyl) -benzamid
   3.1g (10mMol) der Zwischenverbindung 4a wurden analog Beispiel 2c mit der Verbindung 13b umgesetzt, wobei man 1.9g Produkt erhielt.
b) (S)-(1-N(3-Morpholin-1-yl-3-propan-1-yl)carbamoyl-1-oxo-3-phenyl-propan-2-yl)-4-(napth-2-ylamido)methyl-benzamid
   1.2g (2mMol) der Zwischenverbindung 14a wurden analog Beispiel 1f oxidiert. Ausbeute: 0.83g (73%).
   1H-NMR (D₆-DMSO) : δ = 1.6(2H), 2.2(6H), 3.0(1H), 3-3.2(3H), 3.5(4H), 4.6(2H), 5.2(1H), 6.9-8.0(16H), 8.4(1H), 8.8(1H) und 9.0(1H) ppm.

### Beispiel 15

### (S)-N(1-N(3-Morpholin-1-yl-propan-1-yl)-carbamoyl-1-oxo-3-phenylpropan-2-yl)-2-phenyl-benzamid

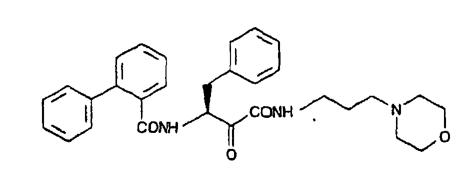

a) N(2(S)-1-N(3-Morpholin-1-yl-(propan-1-yl)carbamoyl-1-hydroxy-3-phenyl-propan-2-yl)-2-phenyl-benzamid
   2g (10mMol) Biphenyl-2-carbonsäure wurden analog Beispiel 2c mit der Zwischenverbindung 13b umgesetzt, wobei man 1.8g Produkt erhielt.
b) (S)-N(1-N(3-Morpholin-l-yl-propan-l-yl)carbamoyl-l-oxo-3-phenyl-propan-2-yl)-2-phenyl-benzamid
   1.0g (2mMol) der Zwischenverbindung 15a wurden analog Beispiel 1f oxidiert. Ausbeute: 0.45g (45%).
   1H-NMR (D₆-DMSO) : δ = 1.7(2H), 2.2(6H), 2.8(1H), 3.2(3H), 3.6(4H), 5.2(1H), 7.0-7.8(14H) und 8.9(2H) ppm.

### Beispiel 16

### (S)-2-Methyl-N(1-N(3-morpholin-1-yl-propan-1-yl)carbamoyll-oxo-3-phenyl-propan-2-yl)-5(naphth-2-yl-sulfonamido)-benzamid

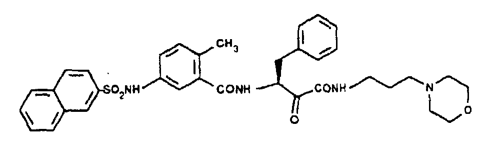

a) 5-Amino-2-methylbenzoesäureethylester
   26.5g (127mMol) 2-Methyl-5-nitrobenzoesäureethylester wurden in Ethanol nach Zugabe von 1g Palladium/Kohle (10%ig) hydriert. Nach dem Filtrieren wurde das Filtrat im Vakuum eingeengt. Ausbeute 0.1g (89%).
b) 2-Methyl-5(naphth-2-ylsulfonamido)-benzoesäureethylester
   12.6g (70.4mMol) der Zwischenverbindung 16a wurden analog Beispiel 7a mit Naphthalin-2-sulfonsäurechlorid bei 0°C umgesetzt, wobei 20.1g Produkt anfielen.
c) 2-Methyl-5(naphth-2-ylsulfonamido)-benzoesäure
   20g (54mMol) der Zwischenverbindung 16b wurden analog Beispiel 8c hydrolysiert, wobei man 15.8g Produkt erhielt.
d) 2-Methyl-N(2(S)-1-N(3-morpholin-1-yl-propan-1-yl)-carbamoyl-l-hydroxy-3-phenyl-propan-2-yl) -5 (naphth-2-ylsulfonamido)-benzamid
   3.4g (10mMol) der Zwischenverbindung 16c wurden analog Beispiel 2c mit der Verbindung 13b umgesetzt. Ausbeute: 3.8g.
e) (S)-2-Methyl-N(1-N(3-morpholin-1-yl-propan-1-yl)carbamoyl-1-oxo-3-phenyl-propan-2-yl)-5-(naphth-2-ylsulfonamido)-benzamid
   0.92g (1.5mMol) der Zwischenverbindung wurden analog Beispiel 1f oxidiert. Ausbeute: 0.3g (32%).
   1H-NMR (D₆-DMSO): δ = 1.6(2H), 2.0(3H), 2.3(3H), 2.8(1H), 3.2(2H), 3.2-3.5(3H), 3.6(4H), 5.2(1H), 6.9-8.1(14H), 8.3(1H), 8.7(1H), 8.9(1H) und 10.4(1H) ppm.

### Beispiel 17

### (S)-N(1-Carbamoyl-1-oxo-3-phenyl-propan-2-yl)-2-methyl-5-(naphth-2-ylsulfonamido)- benzamid

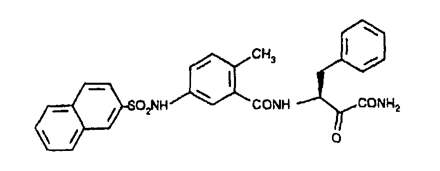

a) N(2(S)-1-Carbamoyl-1-hydroxy-3-phenyl-propan-2-yl)-2-methyl-5(naphth-2-ylsulfon-amido)-benzamid
   2.7g (8mMol) der Zwischenverbindung 16c wurden analog Beispiel 2c mit der Verbindung 2b umgesetzt. Ausbeute: 1.5g (46%).
b) (S)-N(1-Carbamoyl-1-oxo-3-phenyl-propan-2-yl)-2-methyl-5(naphth-2-ylsulfonamido)-benzamid
   1.0g (2mMol) der Zwischenverbindung 17a wurden analog Beispiel 1f oxidiert. Ausbeute: 0.65g (65%).
   1H-NMR (D₆-DMSO): δ = 2.0(3H), 2.8(1H), 3.2(1H), 5.2(1H), 6.8-8.0(15H), 8.2(2H), 8.6(1H) und 10.2(1H) ppm.

### Beispiel 18

### (S)-N(1-Carbamoyl-l-oxo-3-phenyl-propan-2-yl)-4(chinoxalin-2-ylamido)methyl-benzamid

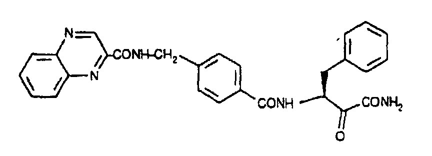

a) N(2(S)-1-Carbamoyl-1-hydroxy-3-phenyl-propan-2-yl)-4(chinoxalin-2-yl-amido)methyl -benzamid
   1.2g (2.7mMol) der Zwischenverbindung 12d wurden analog Beispiel 7a mit Chinoxalin-2-carbonsäurechlorid umgesetzt, wobei 0.8g (62%) Produkt anfielen.
b) (S)-N(l-Carbamoyl-l-oxo-3-phenyl-propan-2-yl)-4(chinoxalin-2-yl-amido)methyl -benzamid
   0.78g (1.6mMol) der Zwischenverbindung 18a wurden analog Beispiel 1f oxidiert. Ausbeute: 0.42g (55%). MS : m/e = 481 (M⁺).

### Beispiel 19

### (S)-N(1-Carbamoyl-1-oxo-3-phenyl-propan-2-yl)-4-(chinolin-4-ylamido)methyl -benzamid

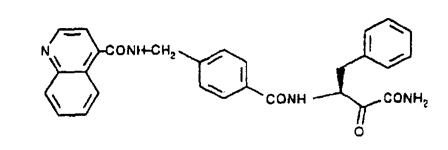

a) N(2(S)-1-Carbamoyl-1-hydroxy-3-phenyl-propan-2-yl) 4-(chinolin-4-yl-amido)methyl -benzamid
   0.8g (1.8mMol) der Zwischenverbindung 12d wurden analog Beispiel 2c mit Chinolin-4-carbonsäure umgesetzt, wobei 0.4g (46%) Produkt anfielen.
b) (S)-N(1-Carbamoyl-1-oxo-3-phenyl-propan-2-yl)-4-(chinolin-4-yl-amido)methyl-benzamid
   0.39g (0.8mMol) der Zwischenverbindung 19a wurden analog Beispiel 1f oxidiert. Ausbeute: 0.27g (70%).
   1H-NMR (D₆-DMSO) : δ = 2.9(1H), 3.1(1H), 4.4(2H), 5.2(1H), 7.0-8.0(15H), 8.8(1H), 8.9(1H) und 9.3(2H) ppm.

### Beispiel 20

### (S)-N(l-Carbamoyl-l-oxo-3-phenyl-propan-2-yl)-4-(chinoxalin-6-ylamido)methyl-benzamid

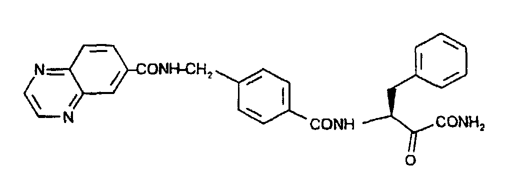

a) N(2(S)-1-Carbamoyl-1-hydroxy-3-phenyl-propan-2-yl)-4-(chinoxalin-6-yl-amido)methyl-benzamid
   0.8g (1.8mMol) der Zwischenverbindung 12d wurden analog Beispiel 2c mit Chinoxalin-6-carbonsäure umgesetzt, wobei 0.36g (42%) Produkt anfielen.
b) (S)-N(1-Carbamoyl-1-oxo-3-phenyl-propan-2-yl)-4-(chinoxalin-6-yl-amido)methyl-benzamid
   0.35g (0.72mMol) der Zwischenverbindung 20a wurden analog Beispiel 1f oxidiert. Ausbeute: 0.23g (66%).
   1H-NMR (D₆-DMSO): δ = 2.8(1H), 3.2(1H), 4.6(2H), 5.2(1H), 7.0-8.2(10H) , 8.7(1H), 8.8(1H), 9.0(2H) und 9.4(2H) ppm.

### Beispiel 21

### (S)-N(1-Carbamoyl-1-oxo-3-phenyl-propan-2-yl)-4-(chinolin-6-ylamido)methyl-benzamid

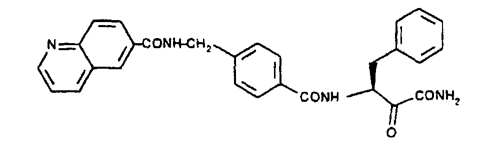

a) N(2(S)-1-Carbamoyl-1-hydroxy-3-phenyl-propan-2-yl)-4-(chinolin-6-yl-amido)methyl-benzamid
   0.8g (1.8mMol) der Zwischenverbindung 12d wurden analog Beispiel 2c mit Chinolin-6-carbonsäure umgesetzt, wobei 0.41g (47%) Produkt anfielen.
b) (S)-N(1-Carbamoyl-1-oxo-3-phenyl-propan-2-yl)-4-(chinolin-6-yl-amido)methyl-benzamid
   0.4g (0.83mMol) der Zwischenverbindung 21a wurden analog Beispiel 1f oxidiert. Ausbeute 0.34g (85%).
   1H-NMR (D₆-DMSO): δ = 2.9(1H), 3.1(1H), 4.4(2H), 5.2(1H) und 7.0-9.2(19H) ppm.

### Beispiel 22

### (S)-N(1-Carbamoyl-1-oxo-3-phenyl-propan-2-yl)-4-(chinolin-3-ylamido)methyl -benzamid

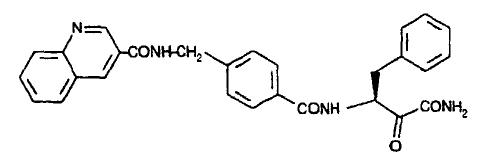

a) N(2(S)-1-Carbamoyl-1-hydroxy-3-phenyl-propan-2-yl)-4-(chinoxalin-3-yl-amido)methyl-benzamid
   1.0g (2.3mMol) der Zwischenverbindung 12d wurden analog Beispiel 2c mit Chinoxalin-3-carbonsäure umgesetzt, wobei 0.89g (80%) Produkt anfielen.
b) (S)-N(1-Carbamoyl-1-oxo-3-phenyl-propan-2-yl)-4-(chinoxalin-6-yl-amido)methyl-benzamid
   0.84g (1.7mMol) der Zwischenverbindung 22a wurden analog Beispiel 1f oxidiert. Ausbeute: 0.75g (90%). MS: m/e = 480 (M⁺).

### Beispiel 23

N(1-Ethoxycarbonyl-1-oxo-3-phenyl-propan-2-yl)-4-(naphth-2-ylamido)-benzamid 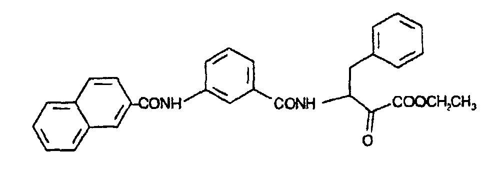
a) 3-(Naphth-2-ylamido)benzoesäure
   14.8 g (0.11Mol) 3-Aminobenzoesäure wurden in 300ml Pyridin gelöst und portionsweise mit 20.6g (0.11Mol) 2-Naphthoylchlorid versetzt. Alles wurde 16h bei Raumtemperatur gerührt. Anschließend wurde alles im Vakuum eingeengt und der Rückstand aus Ethanol umkristallisiert. Ausbeute: 30.3g (97%).
b) N(1-Ethoxycarbonyl-3-phenyl-propan-2-yl)-4-(naphth-2-ylamido)-benzamid
   18.0g(61.8mMol) der Zwischenverbindung 23a und 14.2g (61.8mMol) D,L-Alaninethylester wurden analog Beispiel 2c umgesetzt, wobei man 19.8g (71%) Produkt erhielt.
c) N(1-Carboxy-3-phenyl-propan-2-yl)-4-(naphth-2-yl-amido)-benzamid
   19.5g (41.8mMol) der Zwischenverbindung 23b wurden analog Beispiel 8c hydrolysiert. Ausbeute: 15.2g (83%).
d) N(1-Ethoxycarbonyl-1-oxo-3-phenyl-propan-2-yl)-4-(naphth-2-yl-amido)-benzamid
   Zu einer Lösung aus 14.0g (32mMol) der Zwischenverbindung 23c, 0.4g (3.2mMol) N,N-4-Dimethylaminopyridin und 10.3ml (127.7mMol) Pyridin in 100ml wasserfreiem Tetrahydrofuran wurden 7.1ml (63.9mMol) Oxalsäuremonoethylesterchlorid getropft, so daß die Temperatur auf ca. 40°C stieg. Anschließend wurde alles 3h unter Rückfluß gekocht. Man rührte noch 16h bei Raumtemperatur. Dann gab man vorsichtig 100ml Wasser zu und rührte erneut 30 min. Der Reaktionsansatz wurde mit viel Wasser versetzt und mit Essigester extrahiert. Die organische Phase wurde getrocknet und im Vakuum eingeengt, wobei 17g eines Öles anfielen. Dieses Öl wurde in 100ml absolutem Ethanol gelöst und man fügte 0.24g Kalium-tert.-butanolat zu. Erneut wurde 16h bei Rauamtemperatur gerührt. Der Ansatz wurde im Vakuum eingeengt und der Rückstand chromatographisch (Fließmittel: Methylenchlorid/Essigester = 10/1) gereinigt. Ausbeute: 7.5g (54%).
   1H-NMR (CDCl₃): δ = 1.3(3H), 3.2(1H), 3.3(1H), 4.2(2H), 5.6(1H) und 6.9-8.4(18H) ppm.

### Beispiel 24

### (S)-N(1-Methoxycarbonyl-1-oxo-3-phenyl-propan-2-yl)-2-phenyl-benzamid

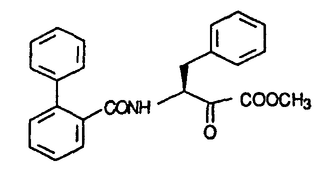

a) N-(3(S)-1-Methoxycarbonyl-1-hydroxy-3-phenyl-propan-2-yl)-2-phenyl-benzamid
   Biphenyl-2-carbonsäure wurde analog Beispiel 2c mit 3(S)-3-Amino-2-hydroxy-4-phenylbuttersäuremethylester umgesetzt.
b) (S)-N(1-Methoxycarbonyl-1-oxo-3-phenyl-propan-2-yl)-2-phenylbenzamid

Die Zwischenverbindung 24a wurde analog Beispiel 1f oxidiert.
MS: m/e = 387 (M⁺).

### Beispiel 25

### (S)-N(N-Carboxymethyl-1-carbamoyl-1-oxo-3-phenyl-propan-2-yl)-3-(2-napthylsulfonamido)-benzamid

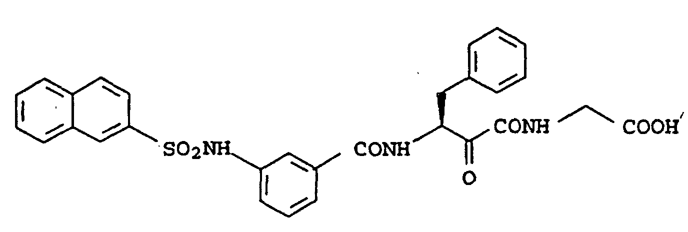

a) O-tert.-Butyl-N(3(S)-1-ethoxycarbonyl-2-hydroxy-4-phenylpropan-2-yl)-urethan
   2.3 g (7.7 mMol) O-tert.-Butyl-N-(3(S)-1-carboxy-2-hydroxy-4-phenylpropan-2-yl)-urethan und 1.1 g (7.7 mmol) Glycinethylesterhydrochlorid wurden analog Beispiel 2c umgesetzt, wobei 1.7 g (57 %) des Produktes erhalten wurden.
b) 3(S)-3-Amino-N-(ethoxycarbonylmethyl)-2-hydroxy-4-phenylbuttersäureamid x Trifluoressigsäure
   1.4 g (3.7 mMol) der Zwischenverbindung 25a wurden in 25 ml Methylenchlorid gelöst und, nachdem man 10 ml Trifluoressigsäure zugegeben hatte, 2h bei Raumtemperatur gerührt. Anschließend wurde alles im Vakuum eingeengt, wobei 1.5 g (100 %) des Produktes anfielen.
c) (S)-N(1-(N-Ethoxycarbonylmethyl-carbamoyl)-1-hydroxy-3-phenyl-propan-2-yl)-3-(2-naphthyl-sulfonamido)-benzamid
   Die Zwischenverbindung 7b wurde analog Beispiel 2c mit dem Produkt 25b umgesetzt. Ausbeute: 1.3 g
d) (S)-N(1-(N-Carboxymethyl-carbamoyl)-1-hydroxy-3-phenyl-propan-2-yl)-3-(2-naphthylsulfonamido)-benzamid
   1.2 g (2mMol) der Zwischenverbindung 25c wurde analog Beispiel 8c mit Lithiumhydroxid hydrolysiert. Ausbeute: 0.77 g (67 %).
e) (S)-N(1-(N-Carboxymethyl-carbamoyl)-1-oxo-3-phenyl-propan-2-yl)-3-(2-naphthylsulfonamido)-benzamid
   0.7 g (1.2 mMol) der Zwischenverbindung 25d wurde analog Beispiel 1f oxidiert, wobei 0.16 g (23 %) des Produktes erhalten wurden. MS: m/e = 559 (M⁺).

### Beispiel 26

N-(1-Carbamoyl-1-oxo-3-phenyl-propan-2-yl)-3-(2-naphthylsulfonamido) -benzamid 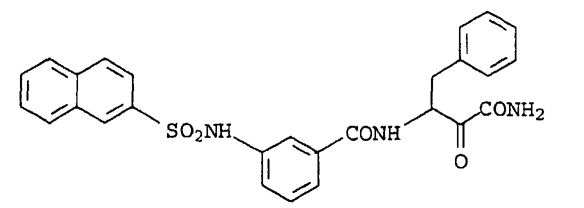
a) Die Zwischenverbindung 7b wurde analog Beispiel 2c mit 3-Amino-2-hydroxy-4-phenylbuttersäureethylester umgesetzt.
b) N(N-Carboxymethyl-1-carbamoyl-1-oxo-3-phenyl-propan-2-yl)-3-(2-naphthylsulfonamido)-benzamid
   Die Zwischenverbindung 26a wurde analog Beispiel 1f oxidiert, wobei das Produkt erhalten wurde.
   ¹H-NMR (D₆-DMSO) : δ = 2.5(2H), 5.2(1H), 7.1-8.1(17H), 8.4(2H), 8.8(1H) und 10.5(1H) ppm.

### Analog lassen sich herstellen:

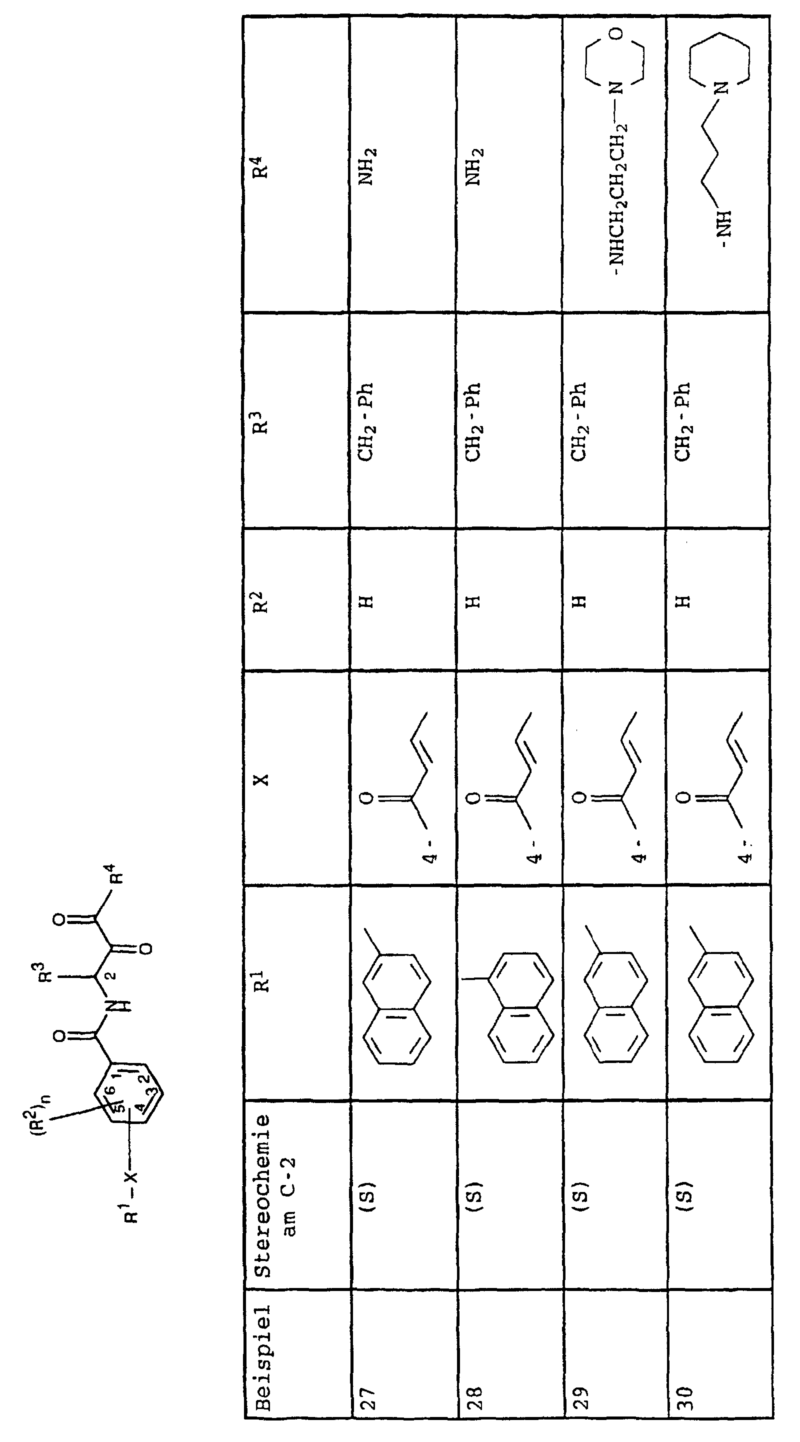

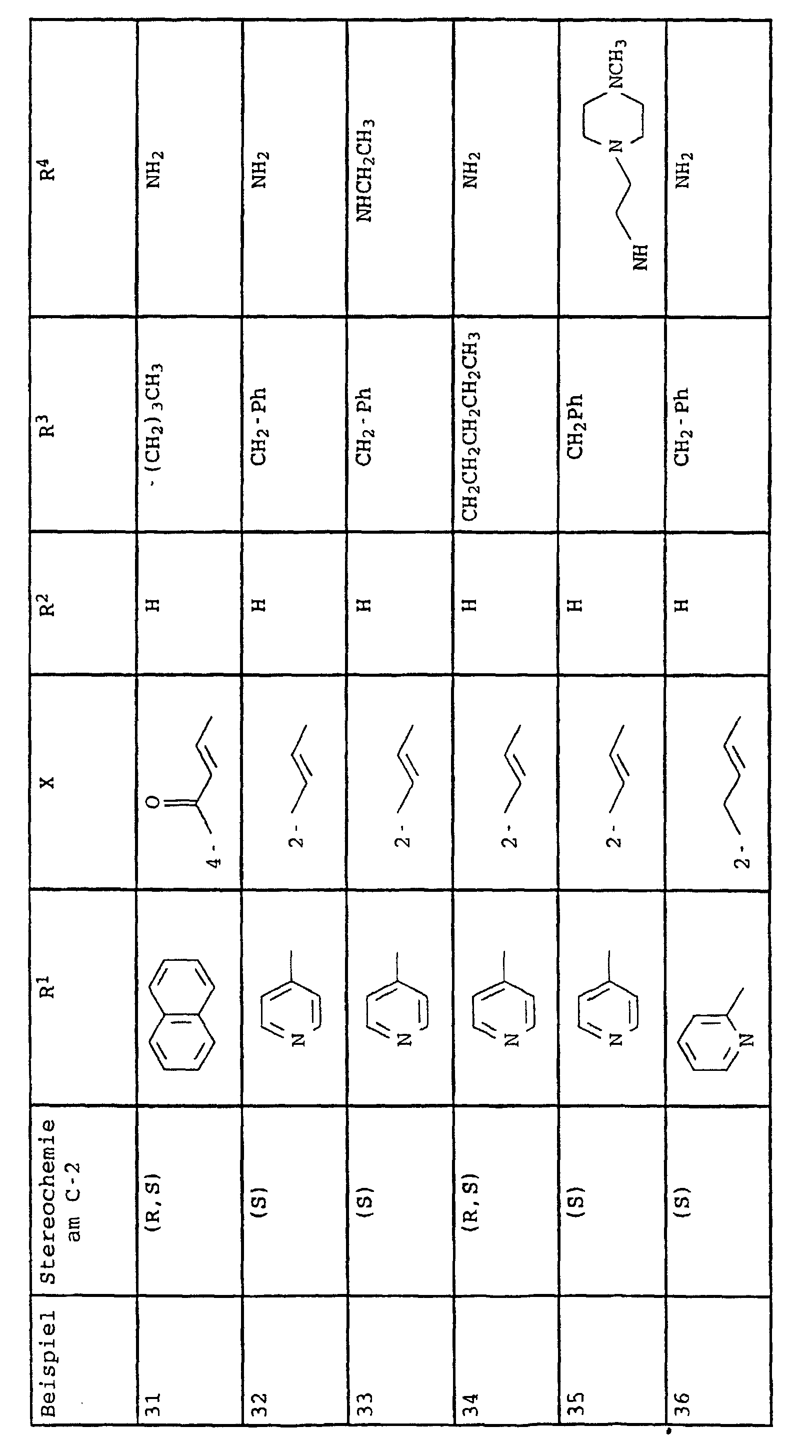

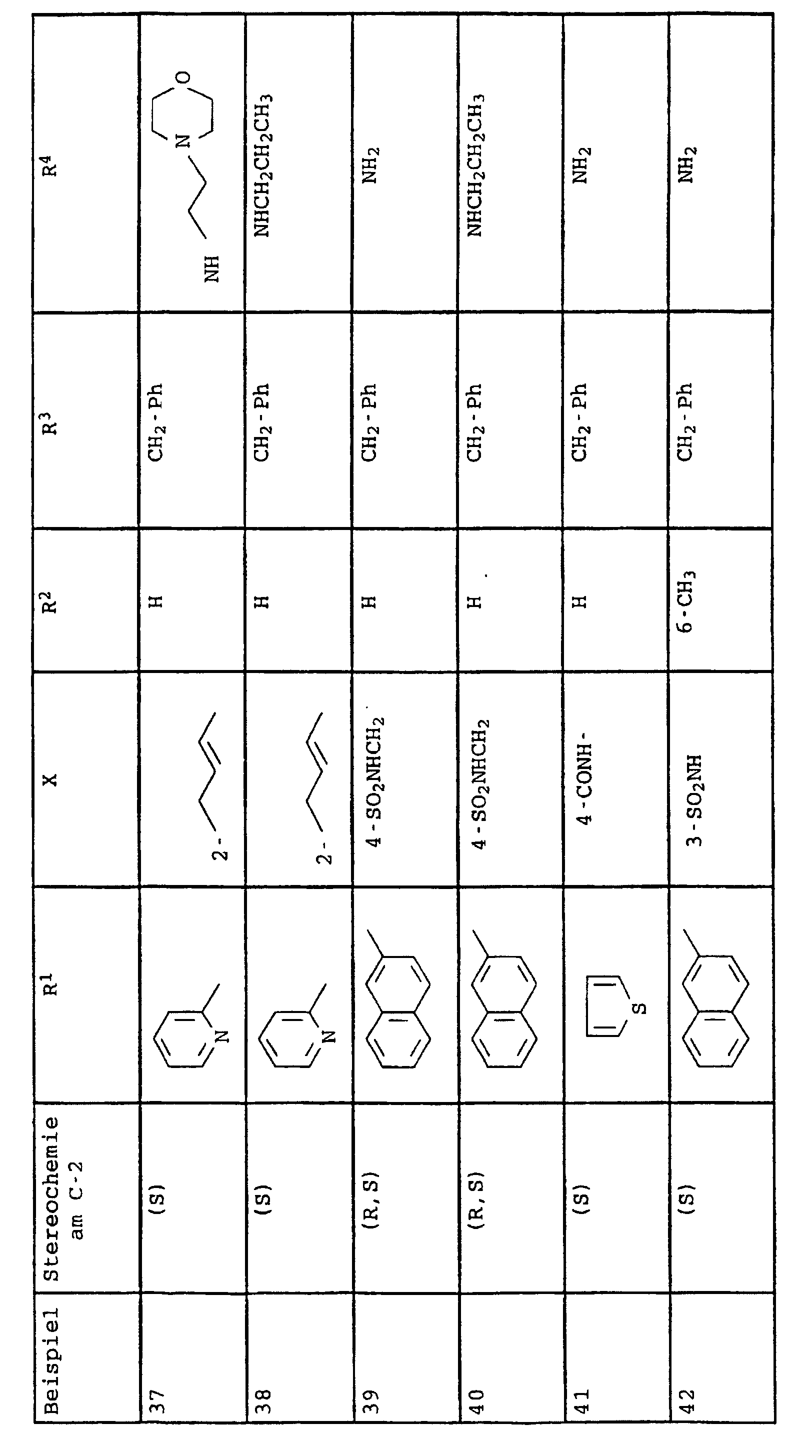

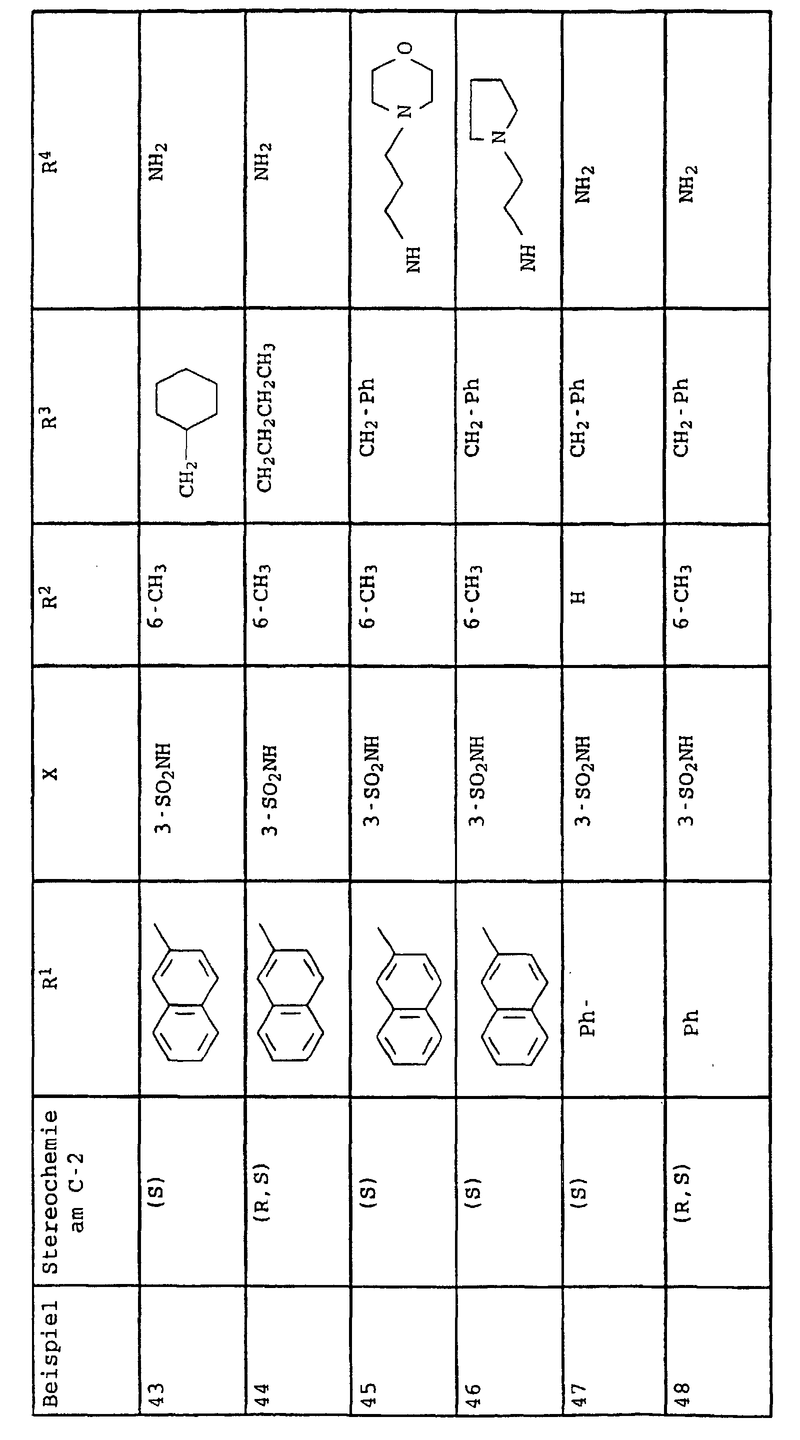

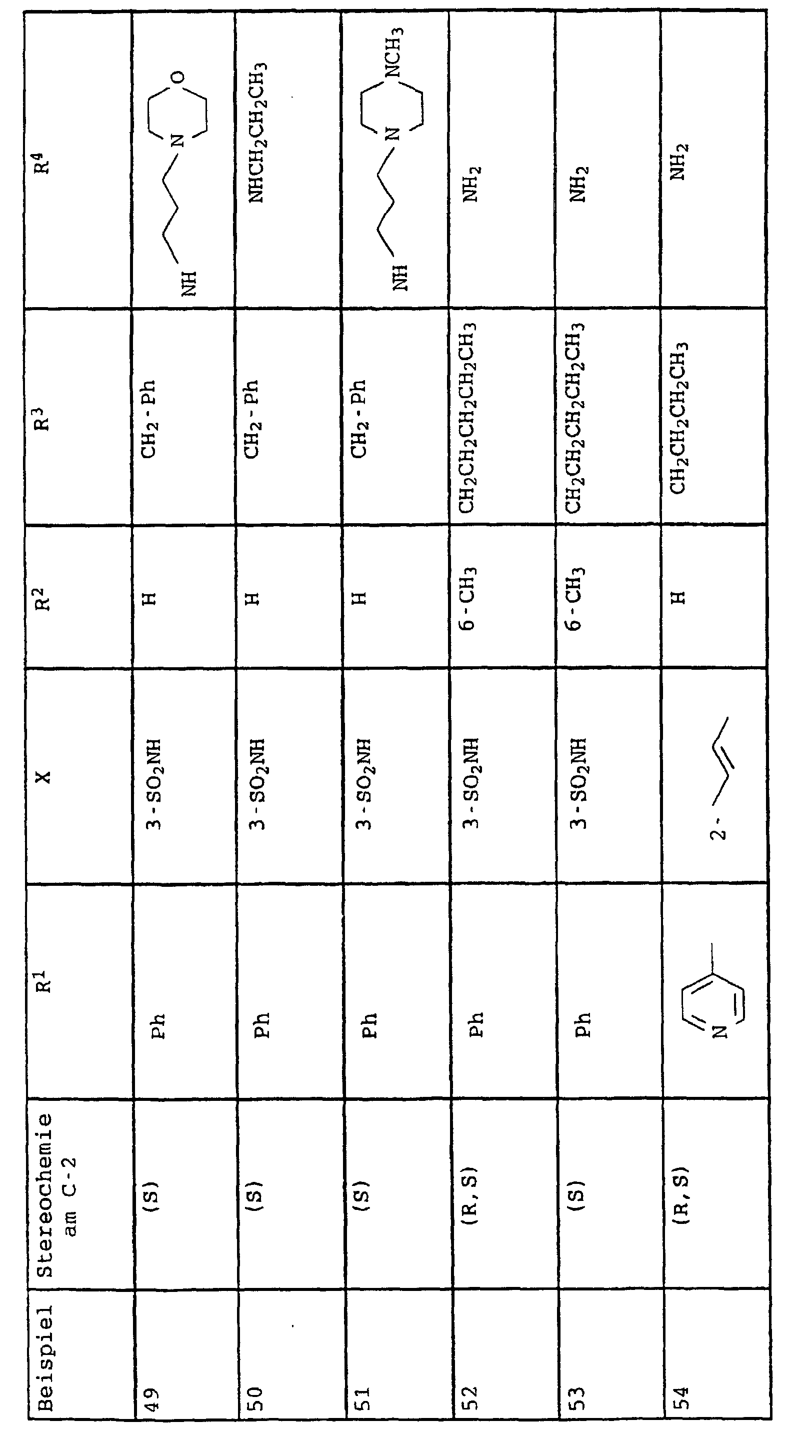

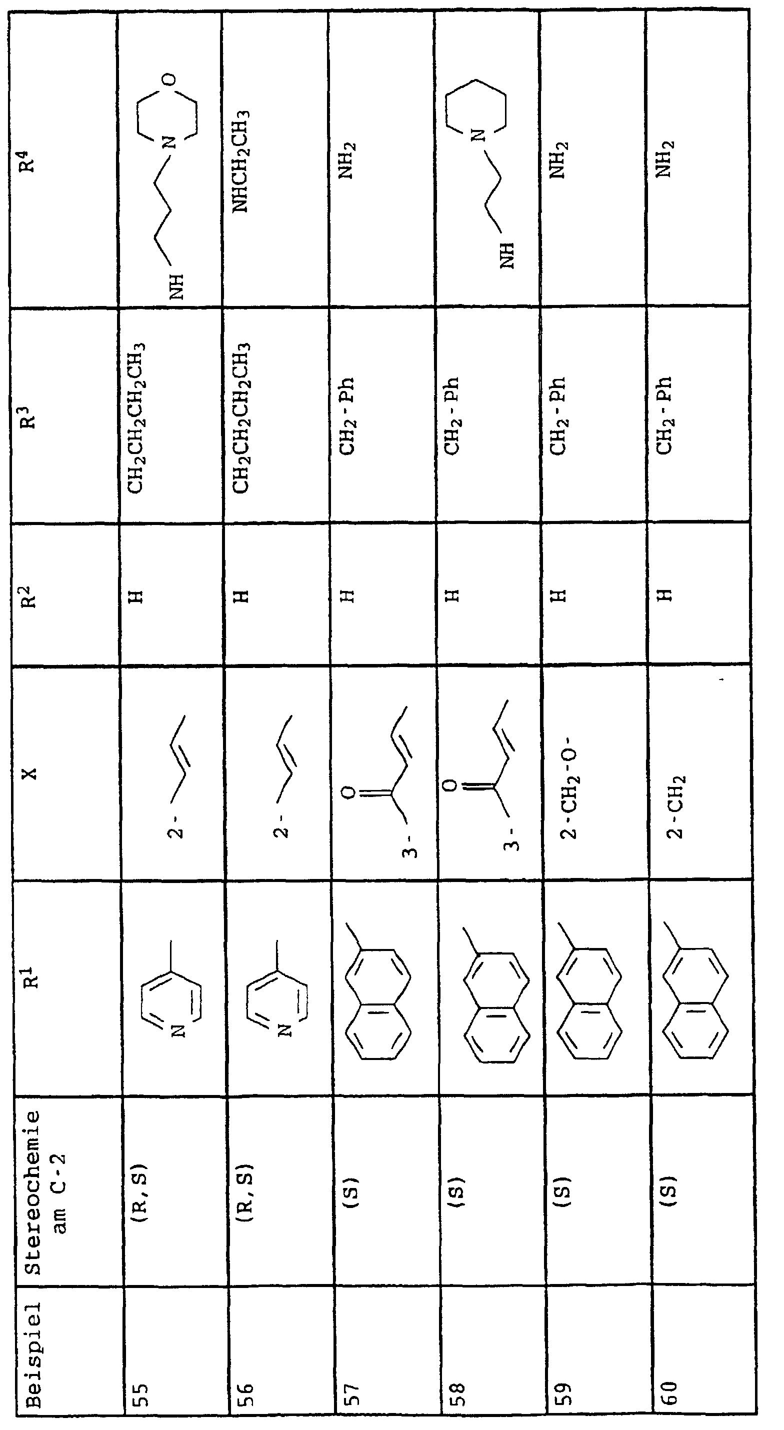

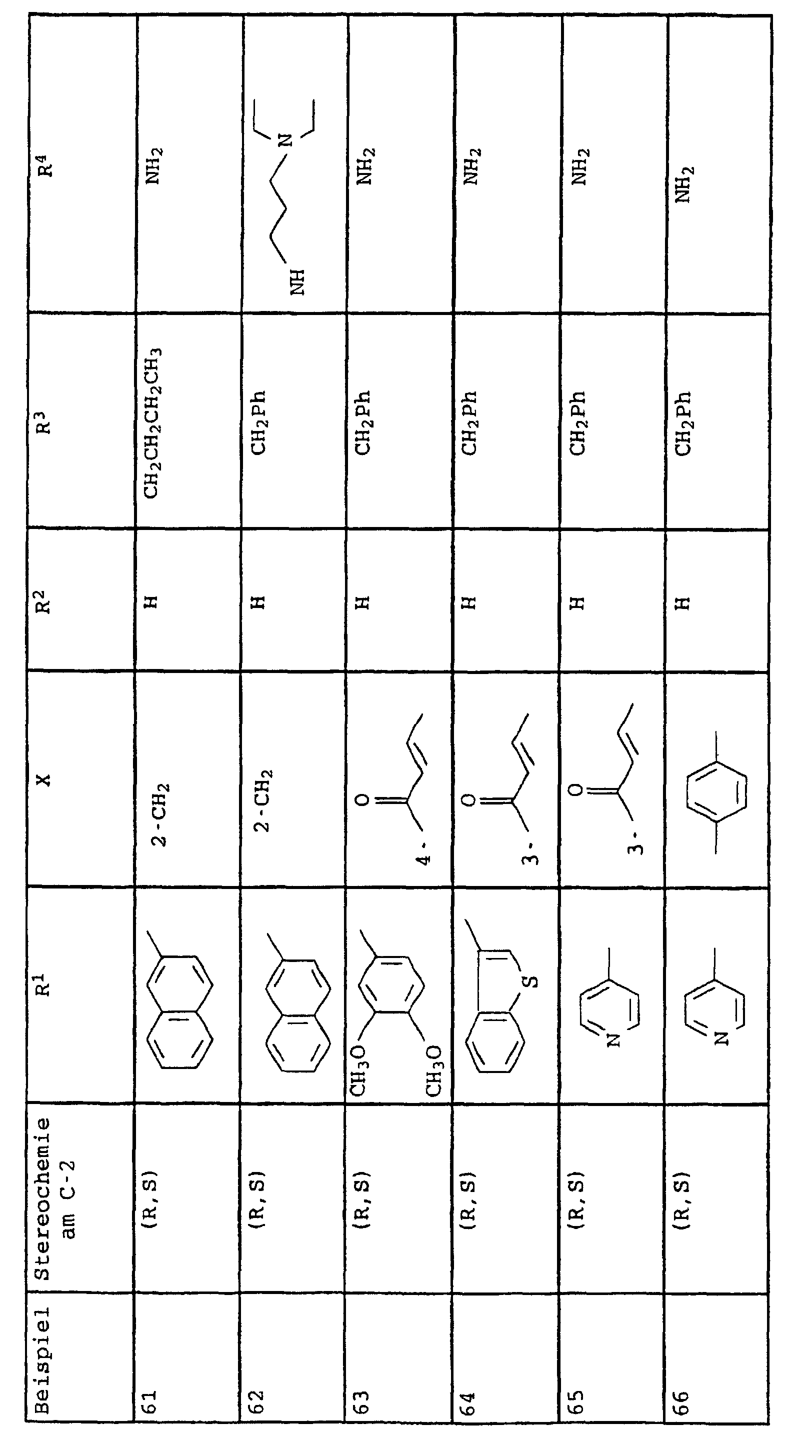

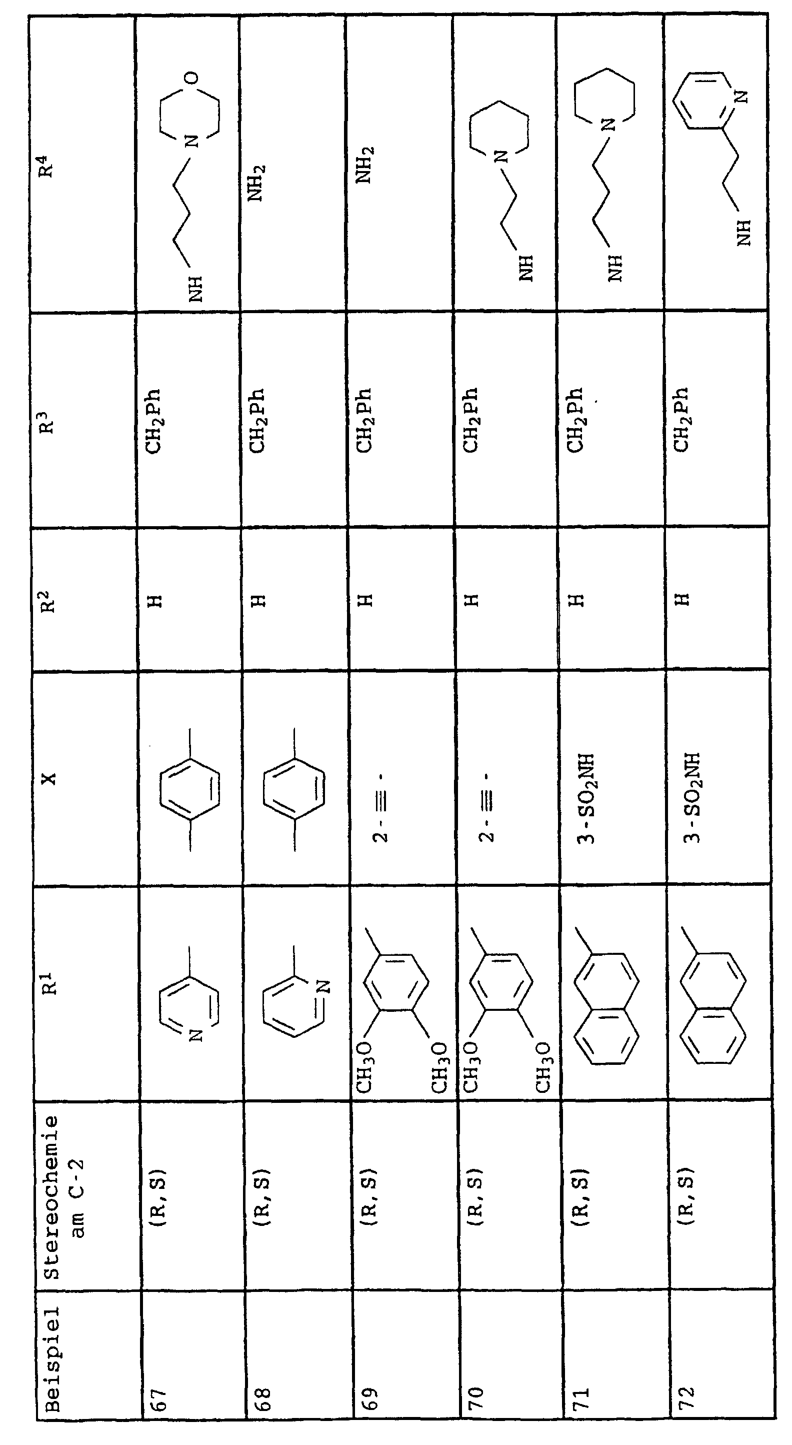

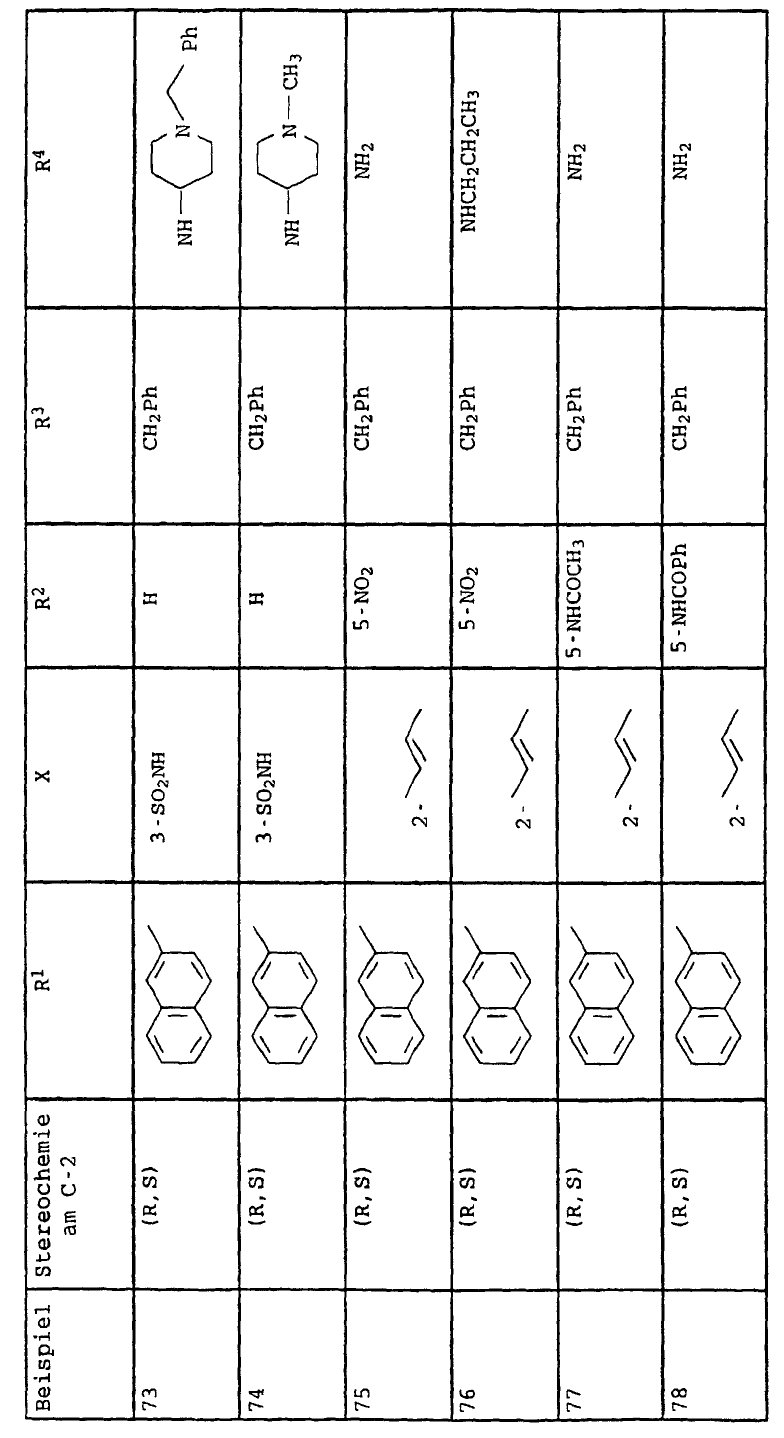

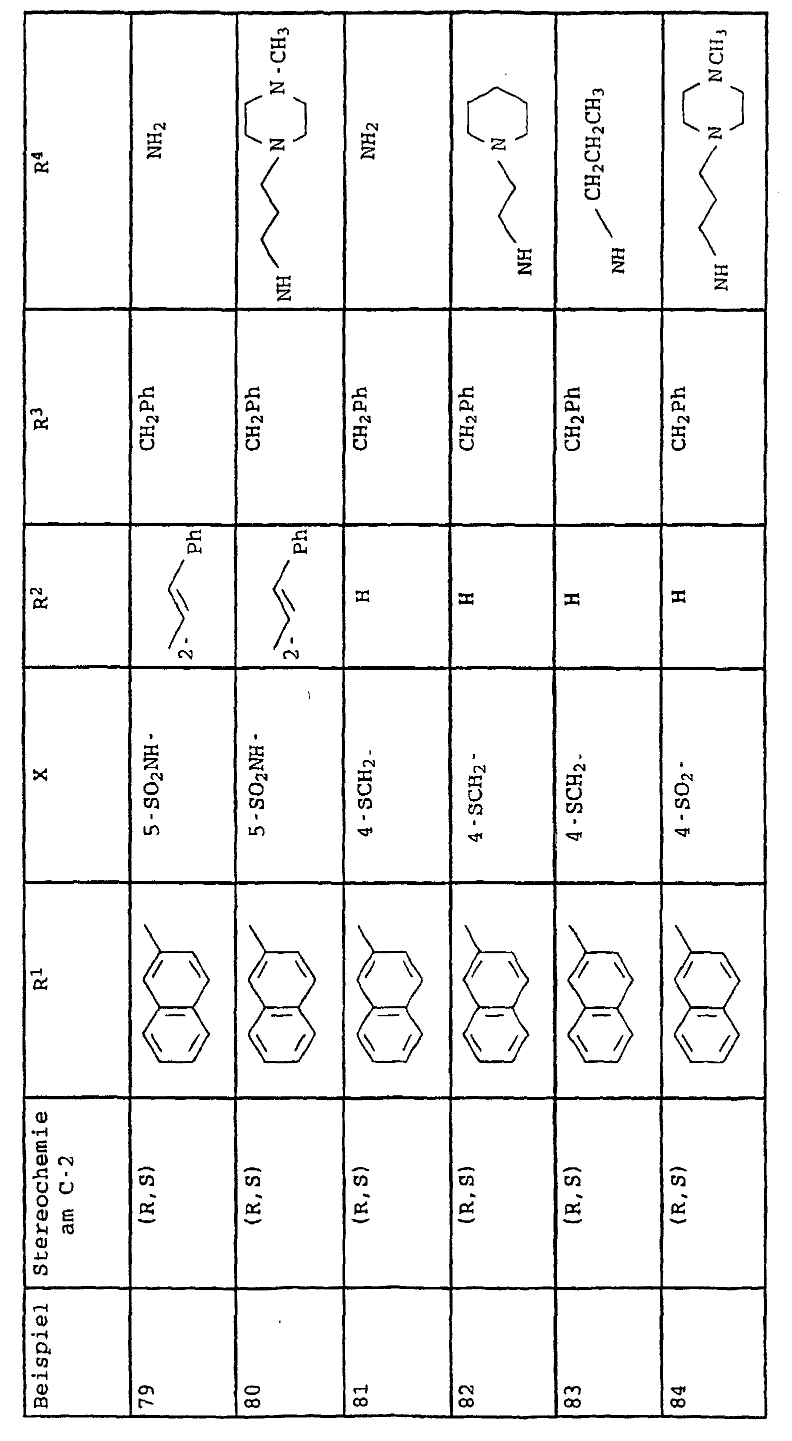

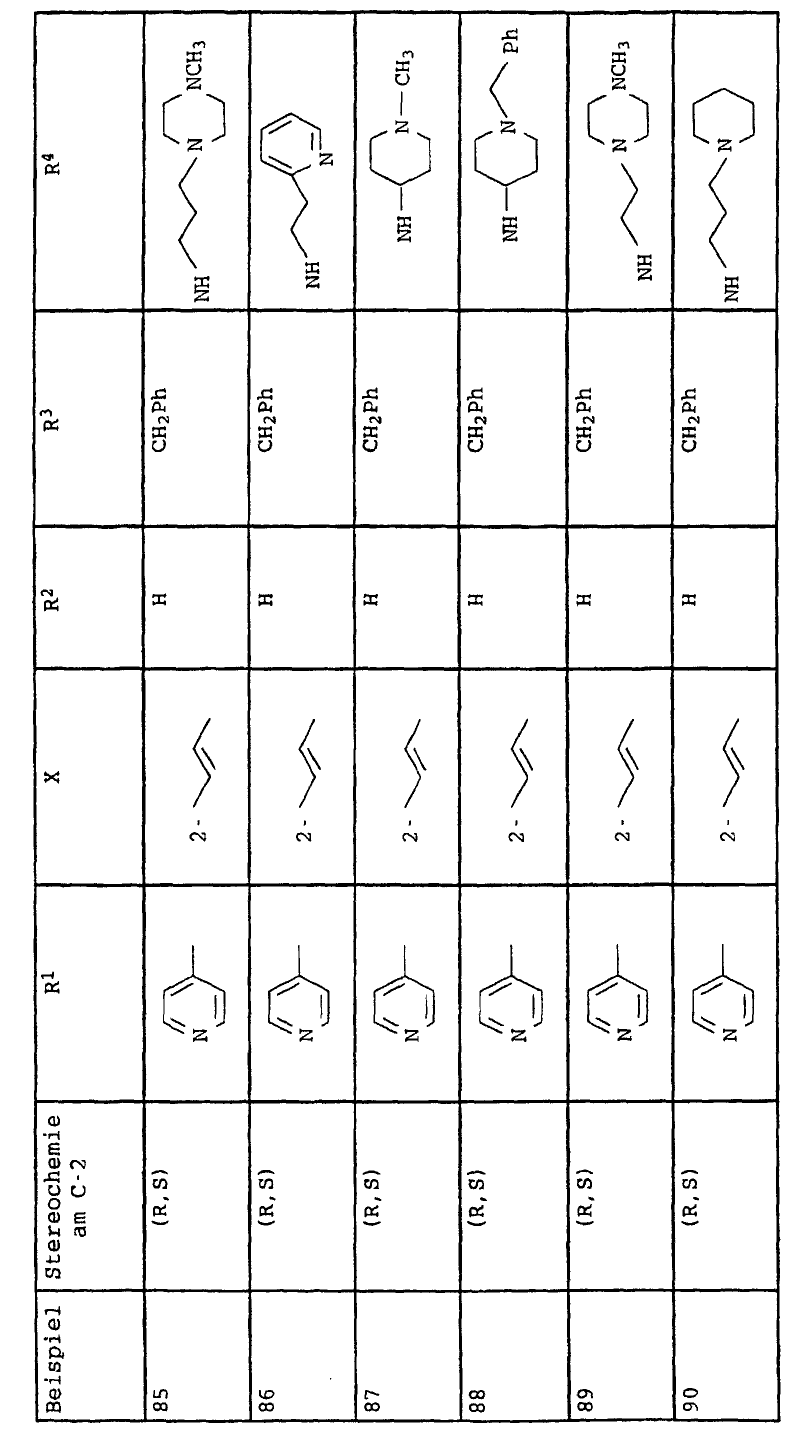

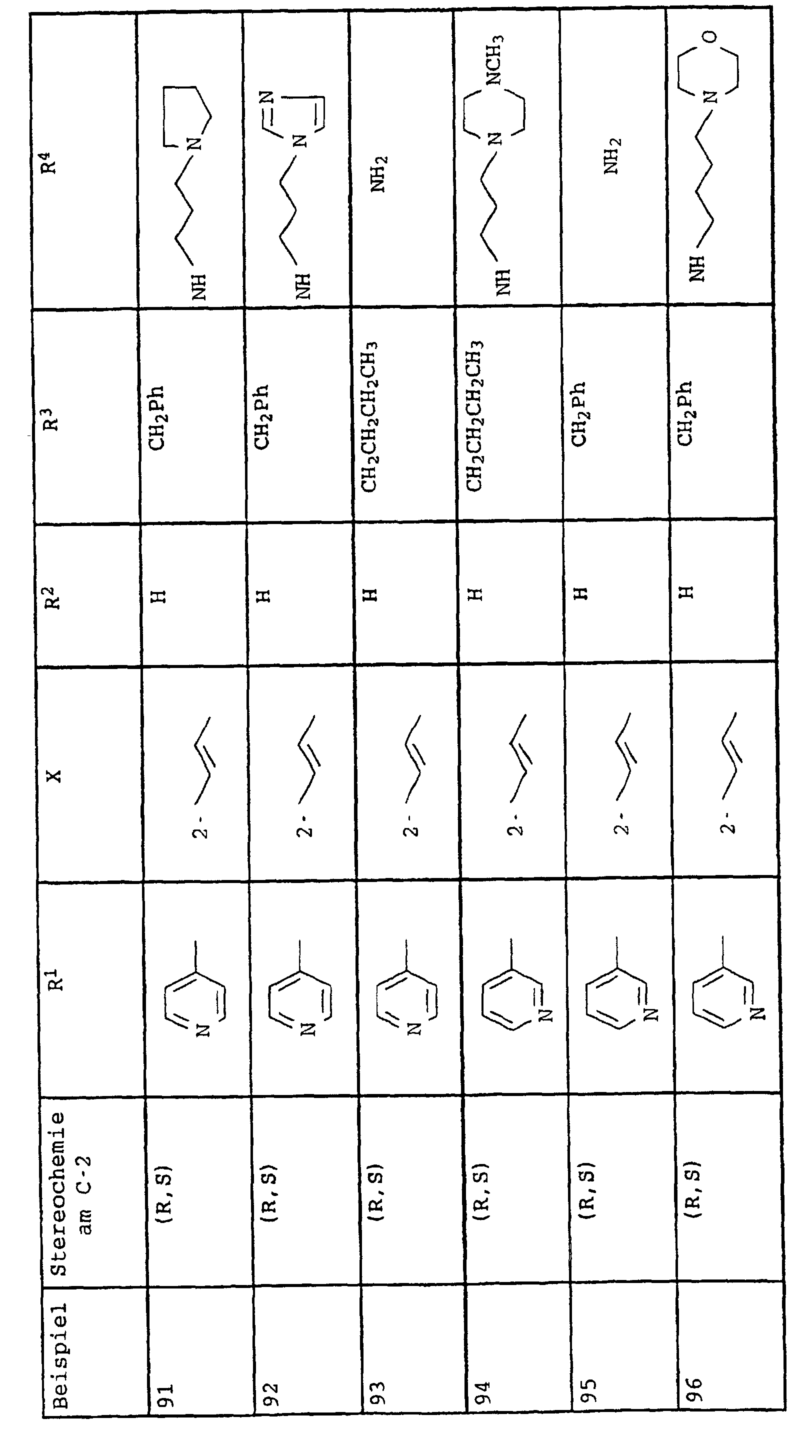

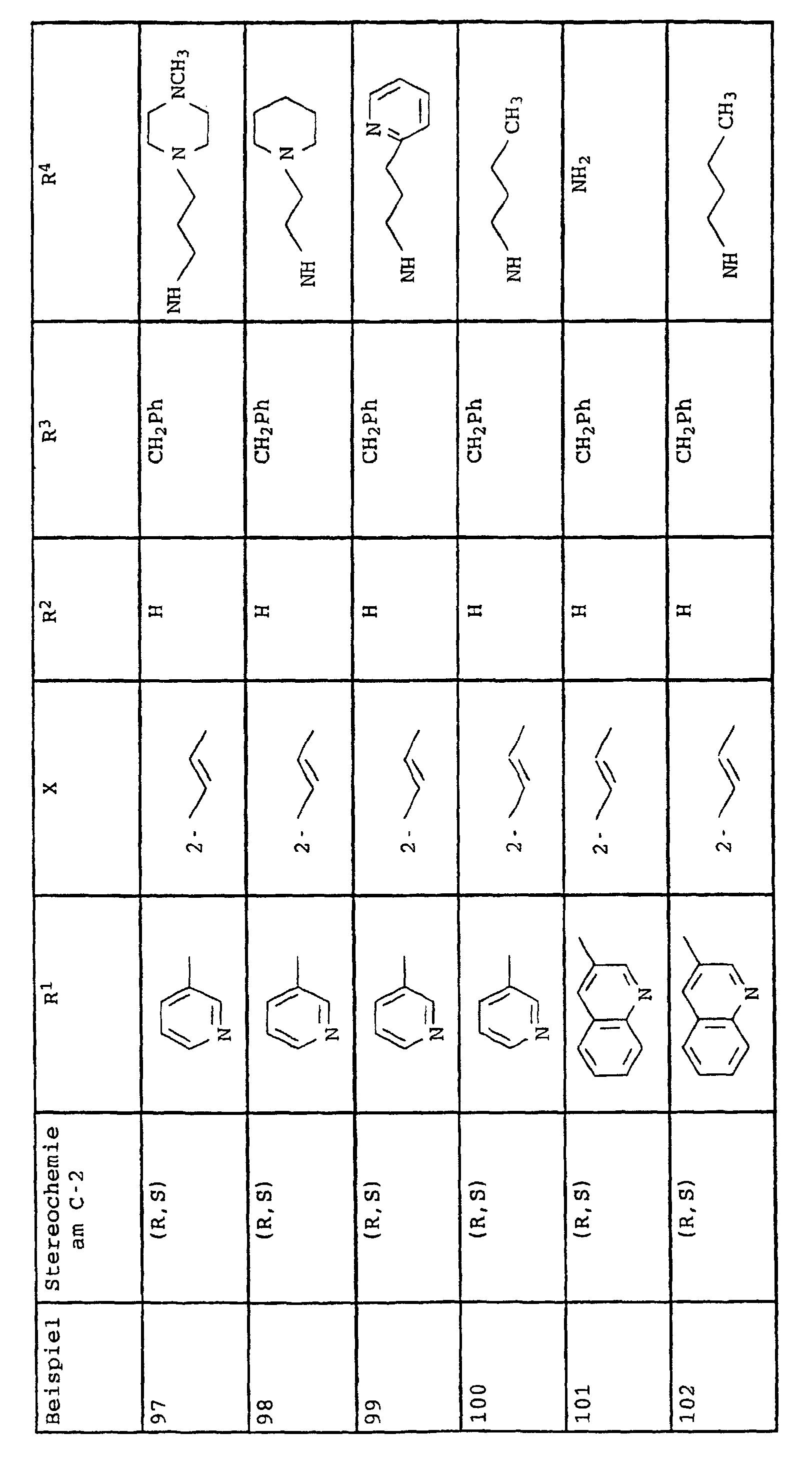

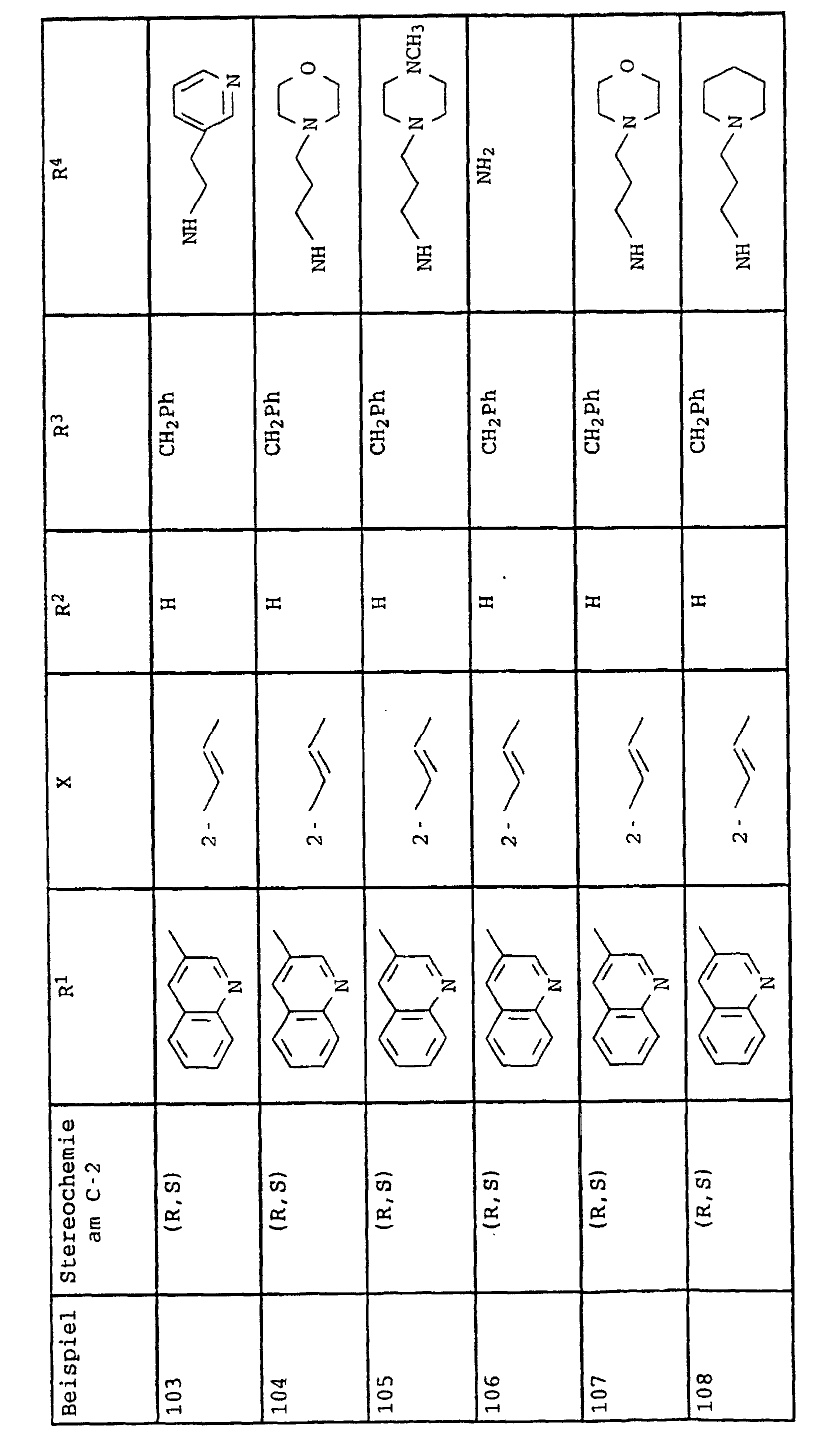

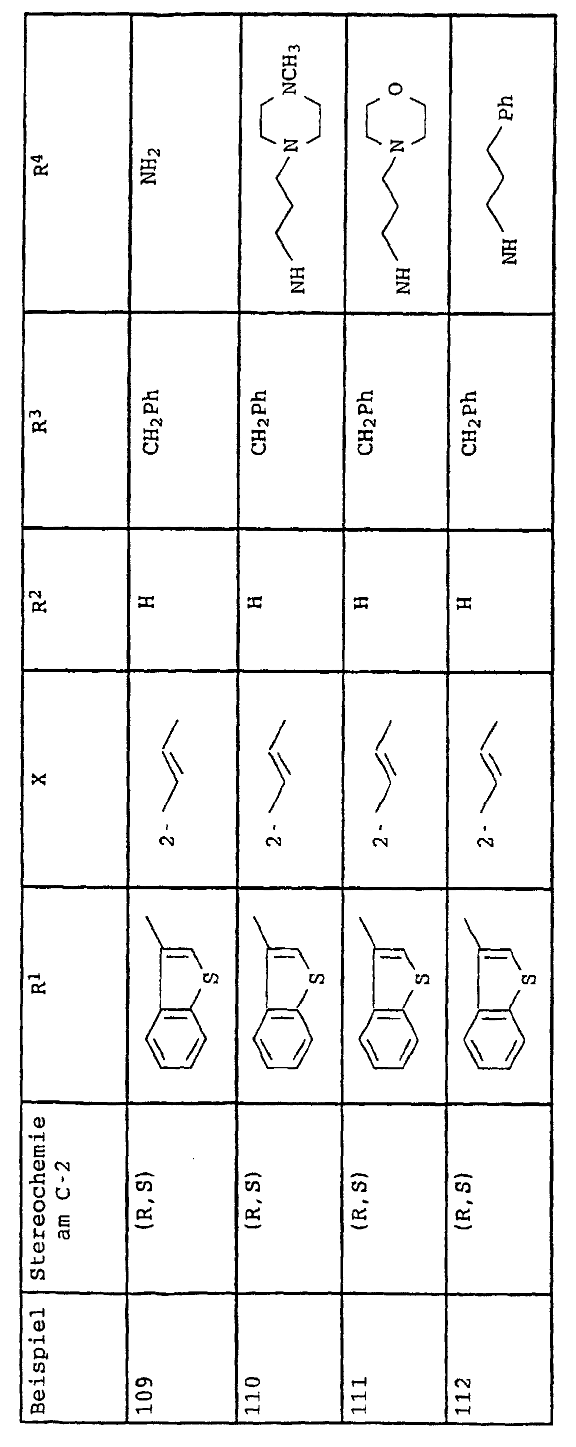

## Patentansprüche

1. Ketobenzamide der Formel I 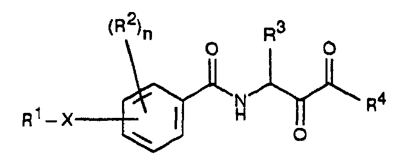 und deren tautomere und isomere Formen sowie gegebenenfalls deren physiologisch verträgliche Salze, worin die Variablen folgende Bedeutung haben:
R¹ Phenyl, Naphthyl, Chinolyl, Pyridyl, Pyrimidyl, Pyrazyl, Pyridazyl, Chinazolyl, Chinoxalyl, Thienyl, Benzothienyl, Benzofuryl,Benzimidazolyl, Furanyl, Indolyl, Isochinolin, Tetrahydroisochinolin oder Tetrahydrochinolin, wobei die aromatischen und heteroaromatischen Ringe noch durch ein, zwei oder drei Reste R⁵ substituiert sein können,
R² Chlor, Brom, Fluor, C₁-C₆-Alkyl, C₂-C₆-Alkenyl, C₂-C₆-Alkinyl, C₁-C₆-Alkyl-Phenyl, C₂-C₆-Alkenyl-Phenyl, C₂-C₆-Alkinyl-Phenyl, Phenyl, NHCO-C₁-C₄-Alkyl, -NHCO-Phenyl, -NHCO-Naphthyl, H₂N-SO₂-C₁₋₄-Alkyl-, COOH, -COO-C₁₋₄-Alkyl, -CONH-C₁₋₄-Alkyl, C₁₋₄-Alkoxy, NO₂, oder NH₂,
R³ C₁-C₆-Alkyl, das noch einen Phenyl-, Cyclopropyl-, Cyclobutyl-, Cyclopentyl-, Cyclohexyl-, Cycloheptyl, Indolyl-, Pyridyl- oder Naphthyl-Ring tragen kann, der seinerseits mit ein oder zwei Resten R⁵ substituiert sein kann,
X eine Bindung, -(CH₂)ₘ-, -(CH₂)ₘ -O-(CH₂)ₒ-, -(CH₂)ₙ-S-(CH₂)ₘ-, -(CH₂)ₙ-SO-(CH₂)ₘ-, -(CH₂)ₙ-SO₂-(CH₂)ₘ -, -CH=CH-, -C≡C-, -CO-CH=CH-, CO-(CH₂)ₘ-, -(CH₂)ₘ -NHCO-(CH₂)ₒ-, -(CH₂)ₘ-CONH-(CH₂)ₒ-, -(CH₂)ₘ -NHSO₂-(CH₂)ₒ-, -NH-CO-CH=CH-, -CH=CH-CO-NH-, - (CH₂)ₘ-SO₂NH-(CH₂)ₒ- oder 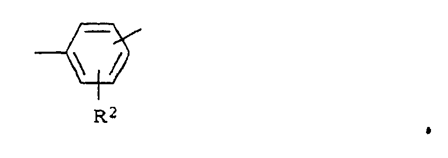
R⁴ OR⁶, NR⁷R⁸, 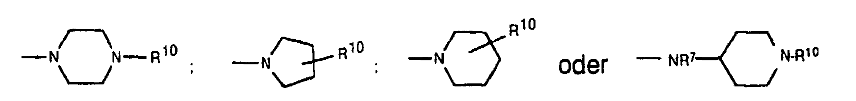
R⁵ Wasserstoff, C₁-C₄-Alkyl, -O-C₁-C₄-Alkyl, OH, Cl, F, Br, J, CF₃, NO₂, NH₂, CN, COOH, COO-C₁-C₄-Alkyl, -NHCO-C₁-C₄-Alkyl, -NHCO-Phenyl, -NHSO₂-C₁-C₄-Alkyl, -NHSO₂-Phenyl, -SO₂-C₁-C₄-Alkyl oder -SO₂-Phenyl,
R⁶ Wasserstoff oder C₁-C₆-Alkyl, das durch einem Phenylring substituiert kann, der selbst noch durch einen oder zwei Reste R⁹ substituiert sein kann,
R⁷ Wasserstoff oder C₁-C₆-Alkyl,
R⁸ Wasserstoff oder C₁-C₆-Alkyl, das noch durch einem Phenylring, der einen oder zwei Reste R⁹ tragen kann, oder einen der Reste 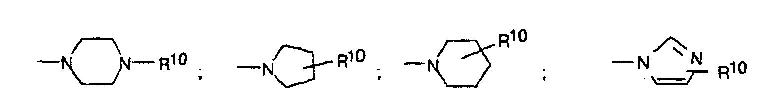 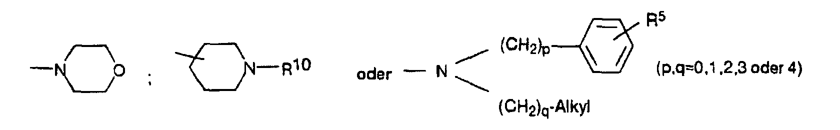 substituiert sein kann,
R⁹ Wasserstoff, C₁-C₄-Alkyl, -O-C₁-C₄-Alkyl, OH Cl, F, Br, J, CF₃, NO₂, NH₂, CN, COOH, COO-C₁-C₄-Alkyl, -NHCO-C₁-C₄-Alkyl, -NHCO-Phenyl, -NHSO₂-C₁-C₄-Alkyl, -NHSO₂-Phenyl, -SO₂-C₁-C₄-Alkyl oder -SO₂-Phenyl,
R¹⁰ Wasserstoff oder C₁-C₆-Alkyl, das durch einen Phenylring substituiert kann, der noch durch einen oder zwei Reste R⁹ substituiert sein kann,
n die Zahl 0, 1 oder 2,
m die Zahl 0, 1, 2, 3 oder 4 und
o die Zahl 0, 1, 2, 3 oder 4.

2. Ketobenzamide der Formel I gemäß Anspruch 1, worin
R² Wasserstoff, C₁-C₄-Alkyl, Fluor oder Chlor,
R³ -CH₂-Phenyl, -CH₂-Cyclohexyl, n-Butanyl oder n-Pentanyl, die jeweils durch einen Rest R⁵ substituiert sein können,
R⁴ -NR⁸ bedeuten und
R¹, X und n die in Anspruch 1 angegebenen Bedeutungen haben.

3. Ketobenzamide der Formel I gemäß Anspruch 1 zur Verwendung bei der Bekämpfung von Krankheiten.

4. Verwendung von Ketobenzamiden der Formel I gemäß Anspruch 1 zur Herstellung von Arzneimitteln, die als Inhibitoren von Cysteinproteasen verwendet werden.

5. Verwendung von Ketobenzamiden der Formel I gemäß Anspruch 1 zur Herstellung von Arzneimitteln zur Behandlung von Krankheiten, bei denen erhöhte Calpain-Aktivitäten auftreten.

6. Verwendung von Ketobenzamiden der Formel I gemäß Anspruch 1 zur Herstellung von Arzneimitteln zur Behandlung von neurodegenerativen Krankheiten und neuronalen Schädigungen.

7. Verwendung von Ketobenzamiden der Formel I gemäß Anspruch 1 zur Herstellung von Arzneimitteln zur Behandlung von Krankheiten und neuronalen Schädigungen, die durch Ischämie, Trauma oder Massenblutungen ausgelöst werden.

8. Verwendung von Ketobenzamiden der Formel I gemäß Anspruch 1 zur Herstellung von Arzneimitteln zur Behandlung von Hirnschlag und Schädel-Hirn-Trauma.

9. Verwendung von Ketobenzamiden der Formel I gemäß Anspruch 1 zur Herstellung von Arzneimitteln zur Behandlung von Alzheimerscher Krankheit und der Huntington-Krankheit.

10. Verwendung von Ketobenzamiden der Formel I gemäß Anspruch 1 zur Herstellung von Arzneimitteln zur Behandlung von Epilepsien.

11. Verwendung der Ketobenzamide der Formel I gemäß Anspruch 1 zur Herstellung von Arzneimitteln zur Behandlung von Schädigungen des Herzens nach cardialen Ischämien, Schädigungen der Nieren nach renalen Ischämien, Sklelettmuskelschädigungen, Muskeldystrophien, Schädigungen, die durch Proliferation der glatten Muskelzellen entstehen, coronarem Vasospasmus, cerebralem Vasospasmus, Katarakten der Augen und Restenosis der Blutbahnen nach Angioplastie.

12. Verwendung der Ketobenzamide der Formel I gemäß Anspruch 1 zur Herstellung von Arzneimitteln zur Behandlung von Tumoren und deren Metastasen.

13. Verwendung der Ketobenzamide der Formel I gemäß Anspruch 1 zur Herstellung von Arzneimitteln zur Behandlung von Krankheiten, bei denen erhöhte Interleukin-1-Spiegel auftreten.

14. Arzneimittelzubereitung enthaltend mindestens ein Ketobenzamid der Formel I gemäß Anspruch 1.

## Claims

1. Ketobenzamides of the formula I 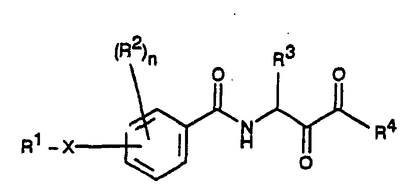 and their tautomeric and isomeric forms, and also, where appropriate, their physiologically tolerated salts, where the variables have the following meanings:
R¹ is phenyl, naphthyl, quinolyl, pyridyl, pyrimidyl, pyrazyl, pyridazyl, quinazolyl, quinoxalyl, thienyl, benzothienyl, benzofuryl, benzimidazolyl, furanyl, indolyl, isoquinoline, tetrahydroisoquinoline or tetrahydroquinoline, where the aromatic and heteroaromatic rings can additionally be substituted by one, two or three R⁵ radicals,
R² is chlorine, bromine, fluorine, C₁-C₆-alkyl, C₂-C₆-alkenyl, C₂-C₆-alkynyl, C₁-C₆-alkyl-phenyl, C₂-C₆-alkenyl-phenyl, C₂-C₆-alkynyl-phenyl, phenyl, NHCO-C₁-C₄-alkyl, -NHCO-phenyl, -NHCO-naphthyl, H₂N-SO₂-C₁₋₄-alkyl-, COOH, -COO-C₁₋₄-alkyl, -CONH-C₁₋₄-alkyl, C₁₋₄-alkoxy, NO₂ or NH₂,
R³ is C₁-C₆-alkyl which can also carry a phenyl, cyclopropyl, cyclobutyl, cyclopentyl, cyclohexyl, cycloheptyl, indolyl, pyridyl or naphthyl ring which, for its part, can be substituted by one or two R⁵ radicals,
X is a bond, -(CH₂)ₘ-, (CH₂)ₘ-O-(CH₂)ₒ-, -(CH₂)ₙ-S-(CH₂)ₘ-, -(CH₂)ₙ-SO-(CH₂)ₘ-, -(CH₂)ₙ-SO₂-(CH₂)ₘ-, -CH=CH-, -C≡C-, -CO-CH=CH-, CO-(CH₂)ₘ-, -(CH₂)ₘ-NHCO-(CH₂)ₒ-, -(CH₂)ₘ-CONH-(CH₂)ₒ-, -(CH₂)ₘ-NHSO₂-(CH₂)ₒ-, -NH-CO-CH=CH-, -CH=CH-CO-NH-, -(CH₂)ₘ-SO₂NH-(CH₂)ₒ- or 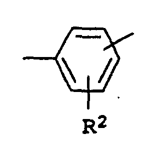
R⁴ is OR⁶, NR⁷R⁸, 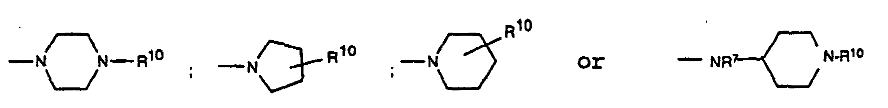
R⁵ is hydrogen, C₁-C₄-alkyl, -O-C₁-C₄-alkyl, OH, Cl, F, Br, I, CF₃, NO₂, NH₂, CN, COOH, COO-C₁-C₄-alkyl, -NHCO-C₁-C₄-alkyl, -NHCO-phenyl, -NHSO₂-C₁-C₄-alkyl, -NHSO₂-phenyl, -SO₂-C₁-C₄-alkyl or -SO₂-phenyl,
R⁶ is hydrogen or C₁-C₆-alkyl which can be substituted by a phenyl ring which, itself, can also be substituted by one or two R⁹ radicals,
R⁷ is hydrogen or C₁-C₆-alkyl,
R⁸ is hydrogen or C₁-C₆-alkyl which can also be substituted by a phenyl ring, which can carry one or two R⁹ radicals, or by one of the radicals 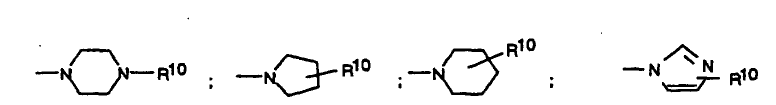 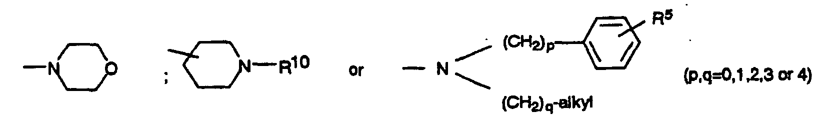
R⁹ is hydrogen, C₁-C₄-alkyl, -O-C₁-C₄-alkyl, OH, Cl, F, Br, I, CF₃, NO₂, NH₂, CH, COOH, COO-C₁-C₄-alkyl, -NHCO-C₁-C₄-alkyl, -NHCO-phenyl, -NHSO₂-C₁-C₄-alkyl, -NHSO₂-phenyl, -SO₂-C₁-C₄-alkyl or -SO₂-phenyl,
R¹⁰ is hydrogen or C₁-C₆-alkyl which can be substituted by a phenyl ring which can also be substituted by one or two R⁹ radicals,
n is the number 0, 1 or 2,
m is the number 0, 1, 2, 3 or 4, and
o is the number 0, 1, 2, 3 or 4.

2. Ketobenzamides of the formula I according to claim 1, where
R² denotes hydrogen, C₁-C₄-alkyl, fluorine or chlorine,
R³ denotes -CH₂-phenyl, -CH₂-cyclohexyl, n-butanyl or n-pentanyl, each of which can be substituted by an R⁵ radical,
R⁴ denotes -NR⁸, and
R¹, X and n have the meanings given in claim 1.

3. Ketobenzamides of the formula I according to claim 1, for use in combating diseases.

4. Use of ketobenzamides of the formula I according to claim 1 for producing drugs which are used as inhibitors of cysteine proteases.

5. Use of ketobenzamides of the formula I according to claim 1 for producing drugs for treating disorders in which increased calpain activities occur.

6. Use of ketobenzamides of the formula I according to claim 1 for producing drugs for treating neurodegenerative disorders and neuronal damage.

7. Use of ketobenzamides of the formula I according to claim 1 for producing drugs for treating diseases and neuronal damage which are induced by ischaemia, trauma or massive haemorrhages.

8. Use of ketobenzamides of the formula I according to claim 1 for producing drugs for treating cerebral stroke and cranial/brain trauma.

9. Use of ketobenzamides of the formula I according to claim 1 for producing drugs for treating Alzheimer's disease and Huntington's disease.

10. Use of ketobenzamides of the formula I according to claim 1 for producing drugs for treating epilepsies.

11. Use of ketobenzamides of the formula I according to claim 1 for producing drugs for treating damage to the heart following cardiac ischaemia, damage to the kidneys following renal ischaemia, skeletal muscle damage, muscular dystrophies, damage which arises due to proliferation of smooth muscle cells, coronary vasospasm, cerebral vasospasm, cataracts of the eyes and restenosis of blood vessels following angioplasty.

12. Use of ketobenzamides of the formula I according to claim 1 for producing drugs for treating tumours and their metastases.

13. Use of ketobenzamides of the formula I according to claim 1 for producing drugs for treating disorders in which increased levels of interleukin 1 occur.

14. Drug preparation which comprises at least one ketobenzamide of the formula I according to claim 1.

## Revendications

1. Cétobenzamides de formule I 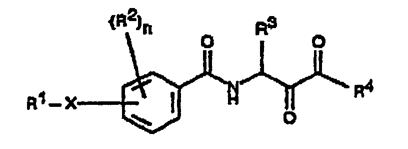 et leurs formes tautomères et isomères, ainsi qu'éventuellement leurs sels physiologiquement acceptables, dans lesquels les variables ont la signification suivante:
R¹ représente un reste phényle, naphtyle, quinoléyle, pyridyle, pyrimidyle, pyrazyle, pyridazyle, quinazolyle, quinoxalyle, thiényle, benzothiényle, benzofuryle, benzimidazolyle, furanyle, indolyle, isoquinoléyle, tétrahydroisoquinoléyle ou tétrahydroquinoléyle, les cycles aromatiques et hétéroaromatiques pouvant encore être substitués par un, deux ou trois restes R⁵;
R² représente un reste chloro, brome, fluoro, alkyle en C₁-C₆, alcényle en C₂-C₆, alcynyle en C₂-C₆, (alkyl en C₁-C₆)phényle, (alcényl en C₂-C₆)phényle, (alcynyl en C₂-C₆)phényle, phényle, -NHCO-(alkyle en C₁-C₄), -NHCO-phényle, -NHCO-naphtyle, H₂N-SO₂-(alkyle en C₁-C₄)-, COOH, -COO-(alkyle en C₁-C₄), -CONH-(alkyle en C₁-C₄), alcoxy en C₁-C₄, NO₂ ou NH₂,
R³ représente un reste allyle en C₁-C₆ pouvant porter en outre un cycle phényle, cyclopropyle, cyclobutyle, cyclopentyle, cyclohexyle, cycloheptyle, indolyle, pyridyle ou naphtyle, qui peut lui-même être substitué par un ou deux restes R⁵,
X représente une liaison ou un groupe -(CH₂)ₘ-, -(CH₂)ₘ-O-(CH₂)ₒ-, -(CH₂)ₙ-S-(CH₂)ₘ-, -(CH₂)ₙ-SO-(CH₂)ₘ-, -(CH₂)ₙ-SO₂-(CH₂)ₘ-, -CH=CH-, -C≡C-, -CO-CH=CH-, -CO-(CH₂)ₘ-, -(CH₂)ₘ-NHCO-(CH₂)ₒ-, -(CH₂)ₘ-CONH-(CH₂)ₒ-, -(CH₂)ₘ-NHSO₂-(CH₂)ₒ-, -NH-CO-CH=CH-, -CH=CH-CO-NH-,-(CH₂)ₘ-SO₂NH-(CH₂)ₒ- ou 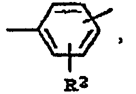
R⁴ représente un reste OR⁶, NT⁷R⁸, 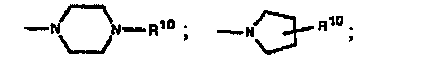 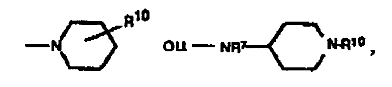
R⁵ représente un atome d'hydrogène ou un reste alkyle en C₁-C₄, -O-alkyle en C₁-C₄, OH, Cl, F, Br, I, CF₃, NO₂, NH₂, CN, COOH, COO-(alkyle en C₁-C₄), -NHCO-(alkyle en C₁-C₄), -NHCO-phényle, -NHSO₂-(alkyle en C₁-C₄), -NHSO₂-phényle, -SO₂-alkyle en C₁-C₄ ou -SO₂-phényle,
R⁶ représente un atome d'hydrogène ou un reste alkyle en C₁-C₄ pouvant être substitué par un cycle phényle qui peut lui-même être encore substitué par un ou deux restes R⁹,
R⁷ représente un atome d'hydrogène ou un reste alkyle en C₁-C₆,
R⁸ représente un atome d'hydrogène ou un reste alkyle en C₁-C₆ qui peut être substitué par un cycle phényle pouvant lui-même être encore substitué par un ou deux restes R⁹, ou par l'un des restes 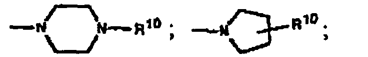 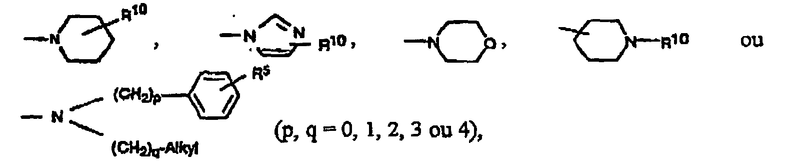
R⁹ représente un atome d'hydrogène ou un reste alkyle en C₁-C₄, -O-alkyle en C₁-C₄, OH, Cl, F, Br, I, CF₃, NO₂, NH₂, CN, COOH, COO-(alkyle en C₁-C₄), -NHCO-(alkyle en C₁-C₄), -NHCO-phényle, -NHSO₂-(alkyle en C₁-C₄), -NHSO₂-phényle, -SO₂-alkyle en C₁-C₄ ou -SO₂-phényle,
R¹⁰ représente un atome d'hydrogène ou un reste alkyle en C₁-C₆ qui peut être substitué par un cycle phényle pouvant lui-même être encore substitué par un ou deux restes R⁹,
n représente le nombre 0, 1 ou 2,
m représente le nombre 0, 1, 2, 3 ou 4 et
o représente le nombre 0, 1, 2, 3 ou 4.

2. Cétobenzamides de formule I selon la revendication 1, dans lesquels
R² représente un atome d'hydrogène ou un reste alkyle en C₁-C₄, fluoro ou chloro,
R³ représente un reste -CH₂-phényle, -CH₂-cyclohexyle, n-butyle ou n-pentyle, pouvant chacun être substitué par un reste R⁵,
R⁴ représente un reste -NR⁸ et
R¹, X et n ont la signification donnée dans la revendication 1.

3. Cétobenzamides de formule 1 selon la revendication 1, à utiliser dans le traitement de maladies.

4. Utilisation de cétobenzamides de formule I selon la revendication 1 pour la préparation de médicaments utilisés comme inhibiteurs de cystéine-protéases.

5. Utilisation de cétobenzamides de formule I selon la revendication 1 pour la préparation de médicaments destinés au traitement de maladies dans lesquelles interviennent des activités élevées de calpaïne.

6. Utilisation de cétobenzamides de formule I selon la revendication 1 pour la préparation de médicaments destinés au traitement de maladies neurodégénératives et de lésions neuronales.

7. Utilisation de cétobenzamides de formula I selon la revendication 1 pour la préparation de médicaments destinés au traitement de maladies et de lésions neuronales déclenchées par une ischémie, un traumatisme ou des hémorragies massives.

8. Utilisation de cétobenzamides de formule I selon la revendication 1 pour la préparation de médicaments destinés au traitement de l'apoplexie cérébrale et de traumatismes crânio-cérébraux.

9. Utilisation de cétobenzamides de formule I selon la revendication 1 pour la préparation de médicaments destinés au traitement de la maladie d'Alzheimer et la maladie de Huntington.

10. Utilisation de cétobenzamides de formule I selon la revendication 1 pour la préparation de médicaments destinés au traitement d'épilepsies.

11. Utilisation de cétobenzamides de formule 1 selon la revendication 1 pour la préparation de médicaments destinés au traitement de lésions cardiaques après une ischémie cardiale, de lésions des reins après une ischémie rénale, de lésions musculo-squelettiques, de dystrophies musculaires, de lésions dues à la prolifération des cellules des muscles lisses, de vasospasmes coronariens, de vasospasmes cérébraux, de la cataracte oculaire et de la resténose des vaisseaux sanguins après une angioplastie.

12. Utilisation de cétobenzamides de formule I selon la revendication 1 pour la préparation de médicaments destinés au traitement de tumeurs et de leurs métastases.

13. Utilisation de cétobeozamides de formule I selon la revendication 1 pour la préparation de médicaments destinés au traitement de maladies dans lesquelles intervient un taux élevé d'interleukine 1.

14. Composition de médicament contenant au moins un cétobenzamide de formule I selon la revendication 1.
